(19) Europäisches Patentamt
European Patent Office
Office européen des brevets

(11) **EP 3 708 653 A1**

(12) **EUROPEAN PATENT APPLICATION**
published in accordance with Art. 153(4) EPC

(43) Date of publication:
16.09.2020 Bulletin 2020/38

(51) Int Cl.:
*C12N 5/077* (2010.01)    *C12Q 1/02* (2006.01)

(21) Application number: 18876465.8

(22) Date of filing: 09.11.2018

(86) International application number:
PCT/JP2018/041659

(87) International publication number:
WO 2019/093477 (16.05.2019 Gazette 2019/20)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 10.11.2017 JP 2017217804

(71) Applicants:
• Toppan Printing Co., Ltd.
Tokyo 110-0016 (JP)
• Osaka University
Suita-shi, Osaka 565-0871 (JP)

(72) Inventors:
• MATSUSAKI, Michiya
Suita-shi
Osaka 565-0871 (JP)
• KITANO, Shiro
Tokyo 110-0016 (JP)
• IRIE, Shinji
Tokyo 110-0016 (JP)
• LOUIS, Fiona
Suita-shi
Osaka 565-0871 (JP)

(74) Representative: TBK
Bavariaring 4-6
80336 München (DE)

(54) **ARTIFICIAL FAT TISSUE AND METHOD FOR PRODUCING SAME, METHOD FOR PRODUCING ARTIFICIAL SKIN AND AGENT FOR FAT CELL CULTURE**

(57) Disclosed is an artificial adipose tissue comprising a three-dimensional tissue comprising: cells comprising an adipocyte; and a fragmented extracellular matrix.

*Fig.1*

SEQUENCE LISTING

<110> TOPPAN PRINTING CO., LTD.
OSAKA UNIVERSITY

<120> ARTIFICIAL ASIPOSE TISSUE, METHOD OF PRODUCING THEREOF, METHOD OF
PRODUCING ARTIFICIAL SKIN AND  CULTURE AGENT OF ADIPOCYTE

<130> FP18-1021-00

<150> JP2017-217804
<151> 2017-11-10

<160> 8

<170> PatentIn version 3.5

<210> 1
<211> 19
<212> DNA
<213> Artificial Sequence

<220>
<223> PPAR-gamma-2 Forward primer

<400> 1
gcgattcctt cactgatac                                                    19


<210> 2
<211> 20
<212> DNA
<213> Artificial Sequence

<220>
<223> PPAR-gamma-2 Reverse primer

<400> 2
gcattatgag acatccccac                                                   20


<210> 3
<211> 20
<212> DNA
<213> Artificial Sequence

<220>
<223> FABP4 (aP2) Forward primer

<400> 3
aaccttagat gggggtgtcc                                                   20


<210> 4
<211> 20
<212> DNA
<213> Artificial Sequence

<220>
<223> FABP4 (aP2) Reverse primer

<400> 4
atgcgaactt cagtccaggt                                                   20

```
<210>    5
<211>    22
<212>    DNA
<213>    Artificial Sequence

<220>
<223>    GLUT-4 Forward primer

<400>    5
ttccaacaga taggctccga ag                                             22


<210>    6
<211>    20
<212>    DNA
<213>    Artificial Sequence

<220>
<223>    GLUT-4 Reverse primer

<400>    6
aagcaccgca gagaacacag                                                20


<210>    7
<211>    19
<212>    DNA
<213>    Artificial Sequence

<220>
<223>    RPII Forward primer

<400>    7
cttcacggtg ctgggcatt                                                 19


<210>    8
<211>    18
<212>    DNA
<213>    Artificial Sequence

<220>
<223>    RPII Reverse primer

<400>    8
gtgcggctgc ttccataa                                                  18
```

## Description

### Technical Field

The present invention relates to an artificial adipose tissue and a method for producing the same, a method for producing an artificial skin, and an agent for adipocyte culture.

### Background Art

*In vitro* long-term culture maintenance of mature adipocytes is one of important issues in the fields of, in particular, cosmetic/pharmaceutical assay or plastic surgery. The culturing period of conventional two-dimensional culture on a flat dish is short, and adipocytes are typically dedifferentiated after one week. A past report shows that uniocular mature adipocytes cannot be maintained over a period of one week or longer (Non Patent Literature 1). In addition, it has been difficult to change a culture medium because substantially more matured adipocytes tend to float over a culture dish due to their intracellular lipid droplets.

To provide a solution to these problems, three-dimensional cell culture technology has attracted attention. WAQAR HASSAN et al. have encapsulated and cultured cells in hydrogel having, as components, a PEG-based copolymer and hyaluronic acid (Non Patent Literature 2). Unfortunately, in this method, the cells are isolated and cultured, and as a result of which contact between the cells is physically difficult and their differentiation efficiency is thus poor.

DAQUINAG et al. have demonstrated that angiogenesis and lipogenesis can be simultaneously simulated using mouse 3T3-L1 preadipocytes and GFP-expressing mouse endothelial cells in a three-dimensionally structured culture system having magnetic nanoparticles as a platform. The coculture of adipocytes and endothelial cells are distinct by immunostaining, but fat vesicles at day 14 are very small and the maturity is low (Non Patent Literature 3).

Meanwhile, regarding an adipocyte-containing skin model, a report shows a skin model using cow collagen type I hydrogel (3 mg/mL) to structure a double layer of fibroblasts and adipose stem cells. However, the culture requires a very long period of from 56 days to 63 days so as to construct the skin model. Besides, the thickness is thinner than that of a human skin structure (Non Patent Literature 4).

In addition, there is a report on a skin model having three layers structured by fibroblasts, mesenchymal stem cells, and adipocytes resuspended in frozen human plasma hydrogel (1 mg/ml fibrinogen). The model construction, however, needs a long-term culturing process over 35 days. On top of that, a human skin structure was not successfully mimicked morphologically, and the skin was very thin (Non Patent Literature 5).

### Citation List

### Non Patent Literature

Non Patent Literature 1: Toda S, Uchihashi K, Aoki S, et al., Organogenesis. 2009 5(2): 50-56
Non Patent Literature 2: WAQAR HASSAN et al., STEM CELL RESEARCH & THERAPY 2013, 21; 4(2): 32
Non Patent Literature 3: DAQUINAG, et al., TISSUE ENGINEERING. PART C, METHODS, 2013 May; 19(5): 336-44.
Non Patent Literature 4: TROTTIER et al., STEM CELLS, 2008 Oct; 26 (10): 2713-23
Non Patent Literature 5: MONFORT et al., J Tissue Eng Regen Med., 2013 Jun; 7(6): 479-90

### Summary of Invention

### Technical Problem

It is an object of one embodiment of the present invention to provide an artificial adipose tissue capable of being maintained for a long period of time and to provide a production method capable of producing the artificial adipose tissue in a short period. Further, it is an object of one embodiment of the present invention to provide an artificial skin including an artificial adipose tissue capable of being maintained for a long period of time and to provide a production method capable of producing the artificial skin in a short period. Furthermore, it is an object of one embodiment of the present invention to provide an agent for adipocyte culture and to provide a method for evaluating skin permeability of a compound by using the artificial skin.

**Solution to Problem**

The present inventors have found that adipocytes, which are floating cells, and a fragmented extracellular matrix are co-cultured to form a three-dimensional tissue; the adipocytes are differentiated in a short period; and an artificial adipose tissue including the three-dimensional tissue formed can be maintained for a long period of time. Further, it has been found that use of the artificial adipose tissue allows for production of an artificial skin having a thickness close to the thickness of human skin.

Specifically, the present invention provides, for instance, the following [1] to [29].

[1] An artificial adipose tissue comprising a three-dimensional tissue comprising: cells comprising an adipocyte; and a fragmented extracellular matrix.

[2] The artificial adipose tissue according to [1], wherein the extracellular matrix comprises collagen.

[3] The artificial adipose tissue according to [1] or [2], wherein content of the extracellular matrix is from 10 wt% to 30 wt% based on the artificial adipose tissue.

[4] The artificial adipose tissue according to any one of [1] to [3], wherein a thickness thereof is from 1 mm to 10 mm.

[5] The artificial adipose tissue according to any one of [1] to [4], wherein a contraction rate thereof during culturing is 20% or less.

[6] The artificial adipose tissue according to any one of [1] to [5], wherein an average length of the fragmented extracellular matrix is from 100 nm to 200 $\mu$m.

[7] The artificial adipose tissue according to any one of [1] to [6], wherein an average size of lipid droplets in the adipocyte is from 20 $\mu$m to 180 $\mu$m.

[8] An artificial skin comprising a first layer and a second layer, wherein the first layer comprises the artificial adipose tissue according to any one of [1] to [7].

[9] The artificial skin according to [8], wherein the second layer comprises fibroblasts.

[10] The artificial skin according to [8] or [9], further comprising a third layer.

[11] The artificial skin according to [10], wherein the third layer comprises keratinocytes.

[12] The artificial skin according to any one of [8] to [11], wherein a thickness thereof is from 0.2 mm to 10 mm.

[13] A method for producing an artificial adipose tissue, comprising:

> (1) contacting cells comprising an adipocyte with a fragmented extracellular matrix in an aqueous medium; and
> (2) culturing the cells contacted with the fragmented extracellular matrix.

[14] The production method according to [13], wherein a period of the culturing in step (2) is from 10 days to 30 days.

[15] The production method according to [13] or [14], further comprising, between step (1) and step (2), a step of subjecting the fragmented extracellular matrix and the cells to precipitate in the aqueous medium.

[16] The production method according to any one of [13] to [15], wherein the extracellular matrix comprises collagen.

[17] The production method according to any one of [13] to [16], wherein an average length of the fragmented extracellular matrix is from 100 nm to 200 $\mu$m.

[18] A method for producing an artificial skin, comprising:

> forming a first layer, comprising
>
> > (1) contacting first cells comprising an adipocyte with a first fragmented extracellular matrix in an aqueous medium, and
> > (2) culturing the first cells contacted with the first fragmented extracellular matrix; and
>
> forming a second layer, comprising (3) further contacting the first layer with second cells and culturing the second cells.

[19] The method for producing an artificial skin according to [18], wherein an average length of the first fragmented extracellular matrix is from 100 nm to 200 $\mu$m.

[20] The production method according to [18] or [19], wherein a period of the culturing in step (2) is from 10 days to 60 days.

[21] The method for producing an artificial skin according to any one of [18] to [20], wherein step (3) is a step comprising contacting the second cells with a second fragmented extracellular matrix on the first layer in an aqueous medium and culturing the second cells.

[22] The method for producing an artificial skin according to [21], wherein the second extracellular matrix comprises collagen.

[23] The method for producing an artificial skin according to any one of [18] to [22], wherein the second cells comprise fibroblasts.

[24] The method for producing an artificial skin according to any one of [18] to [23], further comprising forming a third layer, comprising further contacting the second layer with third cells and culturing the third cells.

[25] The method for producing an artificial skin according to [24], wherein the third cells comprise keratinocytes.

[26] An agent for culturing an adipocyte, comprising a fragmented extracellular matrix.

[27] The agent for culturing an adipocyte according to [26], wherein the fragmented extracellular matrix is collagen having an average length of from 100 nm to 200 μm.

[28] The agent for culturing an adipocyte according to [26] or [27], which is a differentiation promoter.

[29] A method for evaluating skin permeability of a compound, comprising:

provides the artificial skin according to any one of [8] to [12];
contacting the artificial skin with a test compound;
measuring skin permeability of the test compound using the artificial skin contacted with the test compound; and
comparing between the skin permeability of the test compound and a reference value.

## Advantageous Effects of Invention

According to the present invention, there can be provided an artificial adipose tissue capable of being maintained for a long period of time and to provide a production method capable of producing the artificial adipose tissue in a short period. Further, according to the present invention, there can be provided an artificial skin including an artificial adipose tissue capable of being maintained for a long period of time and to provide a production method capable of producing the artificial skin in a short period. Furthermore, according to the present invention, there can be provided an agent for adipocyte culture and to provide a method for evaluating skin permeability of a compound by using the artificial skin.

Moreover, according to the present invention, there can be provided an artificial skin having a thickness close to the thickness of human skin and a production method thereof.

## Brief Description of Drawings

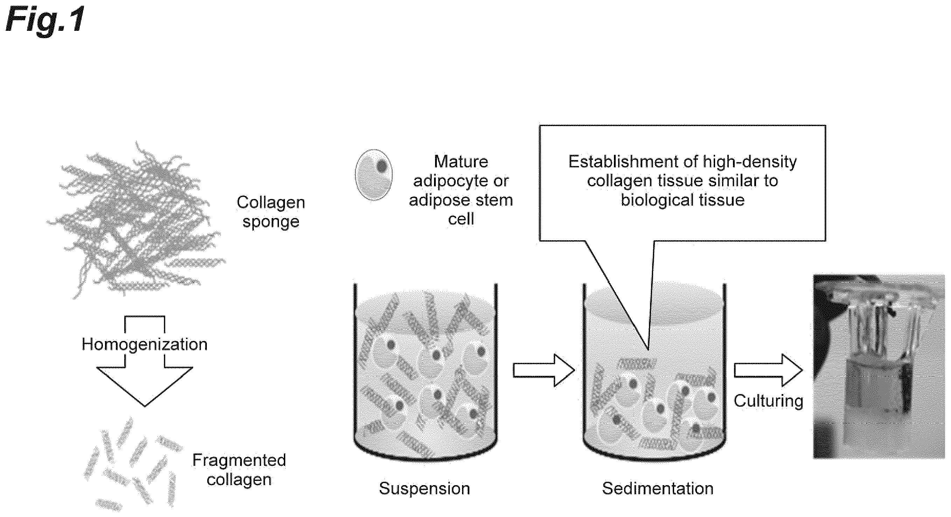

[Figure 1] Figure 1 is a schematic diagram illustrating the steps of producing an artificial adipose tissue including a three-dimensional tissue containing a fragmented collagen and mature adipocytes or adipose stem cells.

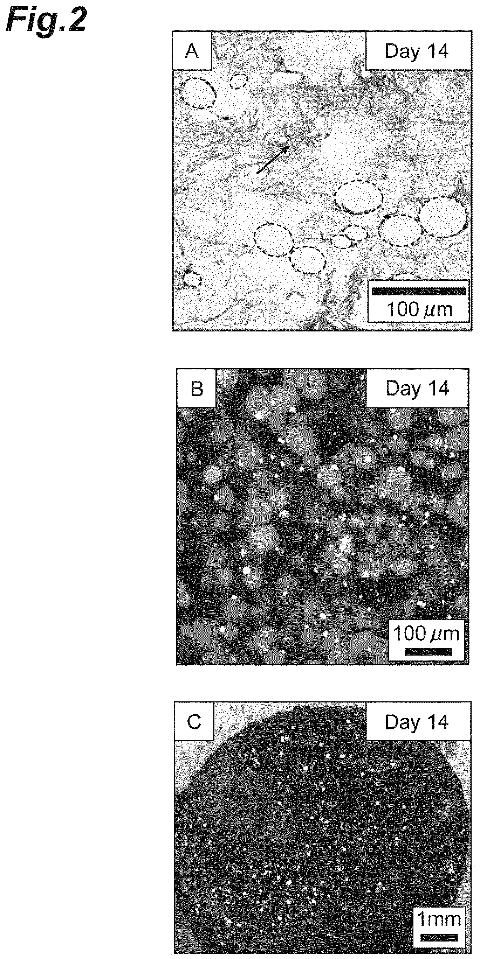

[Figure 2] Figure 2 is photographs showing the results of evaluating an artificial adipose tissue at day 14 of culturing in Example 1. Photograph A shows hematoxylin/eosin (HE) staining results. The arrow indicates a site where fragmented collagen is present. Each dashed line indicates a mature adipocyte. Photograph B shows the results of Nile red lipid staining. White portions indicate nuclei. Photograph C shows the results using a Live/Dead kit. Grey indicates living cells and white indicates dead cells.

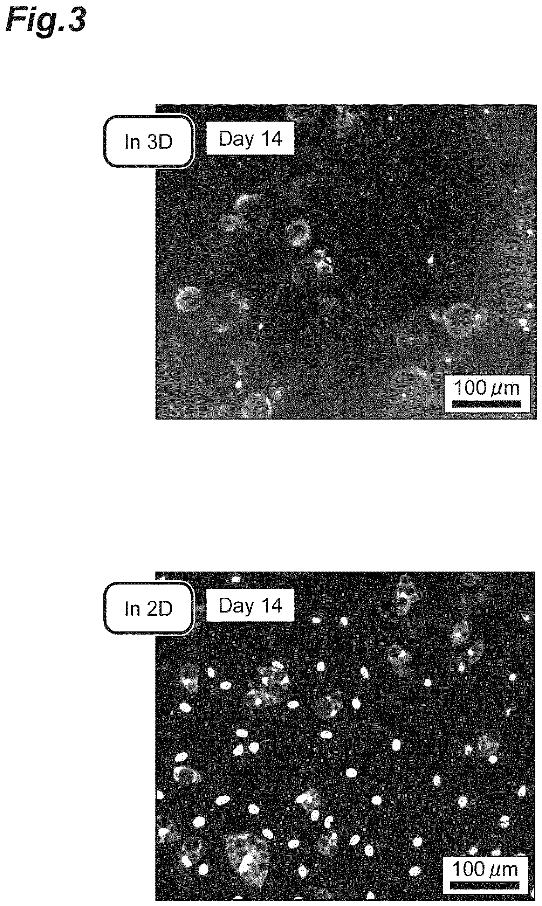

[Figure 3] Figure 3 is photographs showing the anti-perilipin antibody immunostaining results of an artificial adipose tissue in Example 1 and an artificial adipose tissue produced by conventional two-dimensional culture. White portions indicate nuclei. The "In 3D" photograph shows the results of the artificial adipose tissue in Example 1 and the "In 2D" photograph shows the results of the artificial adipose tissue produced by conventional two-dimensional culture.

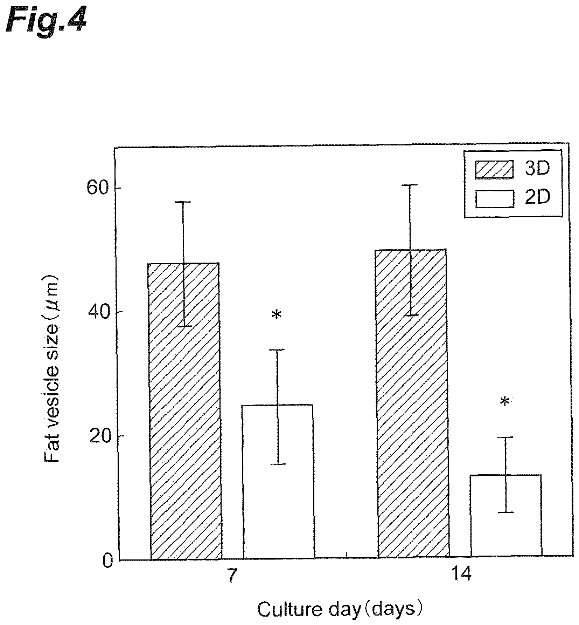

[Figure 4] Figure 4 is a graph showing the results of comparing the fat vesicle size between the artificial adipose tissue in Example 1 and the artificial adipose tissue produced by conventional two-dimensional culture at day 7 of culturing and day 14 (*p < 0.001). In the graph, the "3D" indicates the results of the artificial adipose tissue in Example 1 and the "2D" indicates the results of the artificial adipose tissue produced by conventional two-dimensional culture.

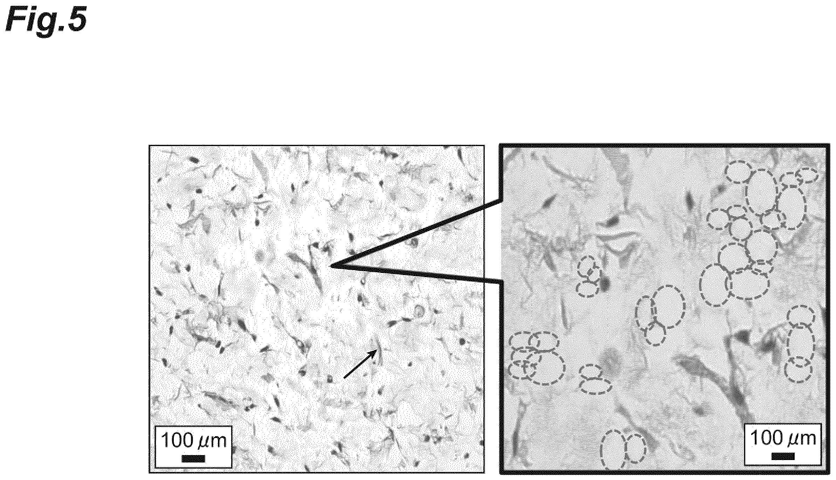

[Figure 5] Figure 5 is photographs showing the HE staining results of an artificial adipose tissue at day 17 of differentiation in Example 2. The upper photograph is a photographic image in which part of the photograph below has been enlarged. Each dashed line indicates a mature adipocyte. The arrow indicates a site where fragmented collagen is present.

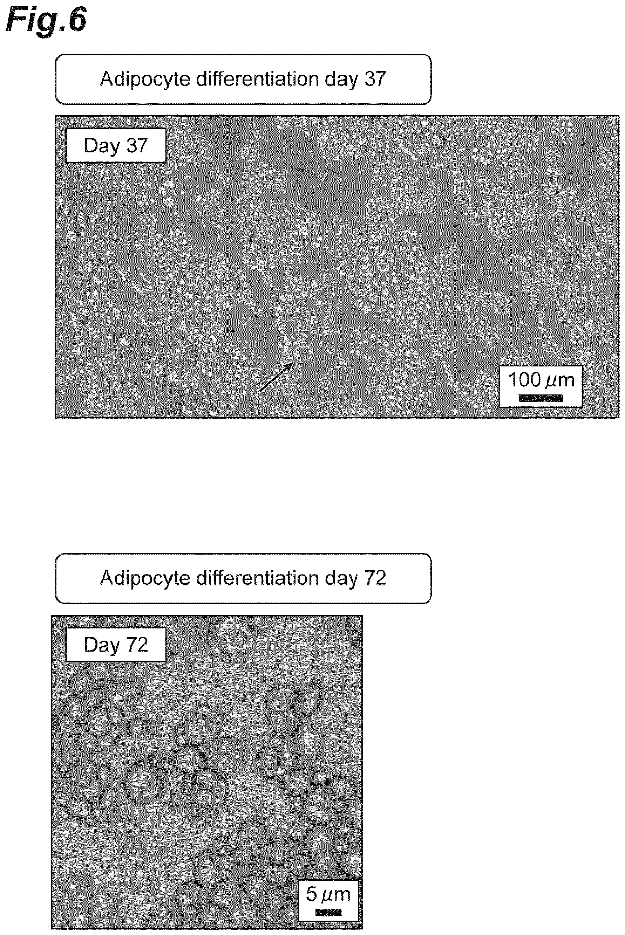

[Figure 6] Figure 6 is electron micrographs of artificial adipose tissues produced by conventional two-dimensional culture at day 37 and day 72 of differentiation. The upper photograph shows an artificial adipose tissue at day 37 of differentiation, and the lower photograph shows an artificial adipose tissue at day 72 of differentiation. The arrow indicates a mature adipocyte.

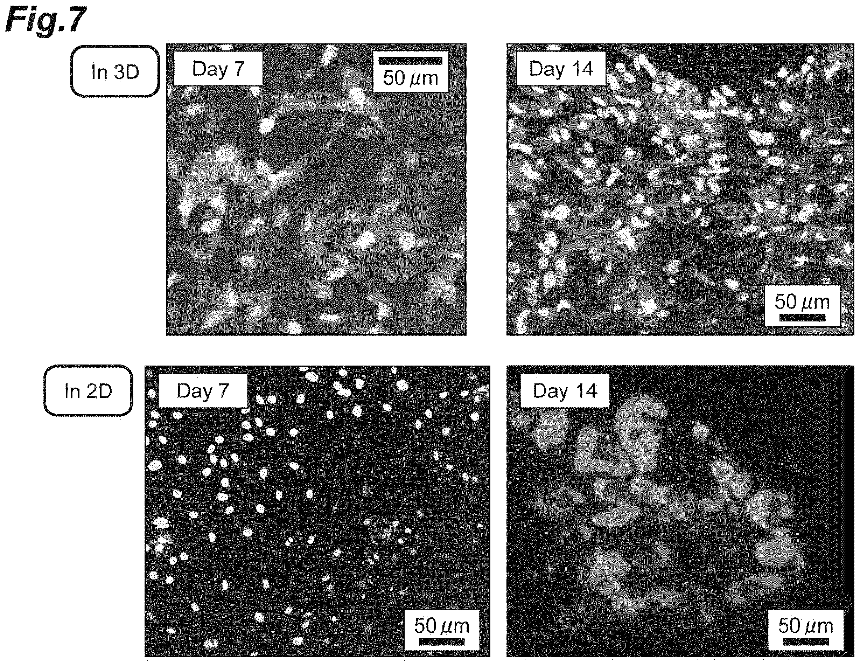

[Figure 7] Figure 7 is photographs showing the anti-perilipin antibody immunostaining results of artificial adipose tissues in Example 2 and artificial adipose tissues produced by conventional two-dimensional culture. White portions indicate nuclei. The "In 3D" photographs show the results of the artificial adipose tissues in Example 2 and the "In 2D" photographs show the results of the artificial adipose tissues produced by conventional two-dimensional culture.

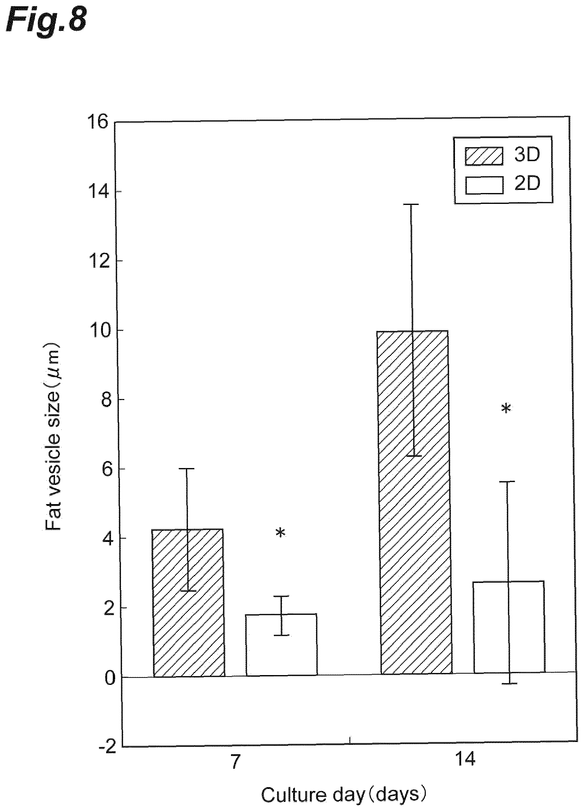

[Figure 8] Figure 8 is a graph showing the results of comparing the fat vesicle size between the artificial adipose tissues in Example 2 and the artificial adipose tissues produced by conventional two-dimensional culture at day 7 of culturing and day 14 (*p < 0.001). In the graph, the "3D" indicates the results of the artificial adipose tissues in Example 2 and the "2D" indicates the results of the artificial adipose tissues produced by conventional two-dimensional culture.

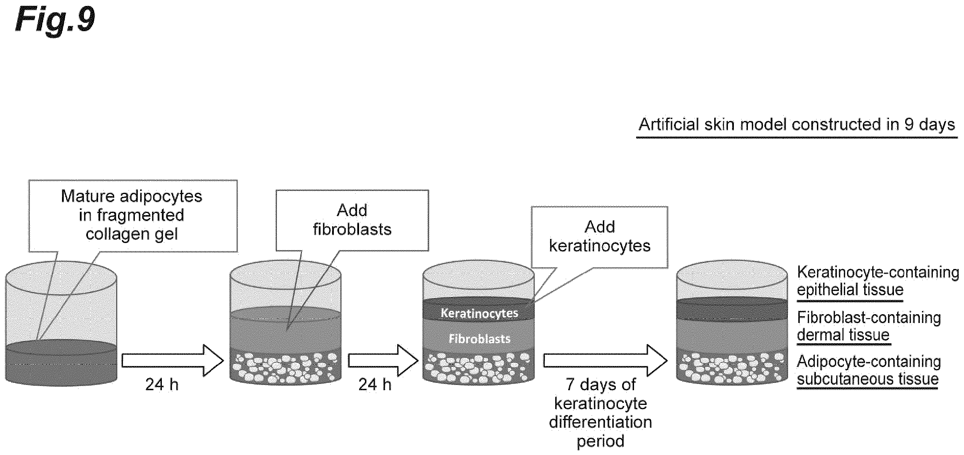

[Figure 9] Figure 9 is a schematic diagram illustrating the steps of producing an artificial skin including: a first layer including an artificial adipose tissue containing a fragmented collagen and mature adipocytes; a second layer containing fibroblasts; and a third layer containing keratinocytes.

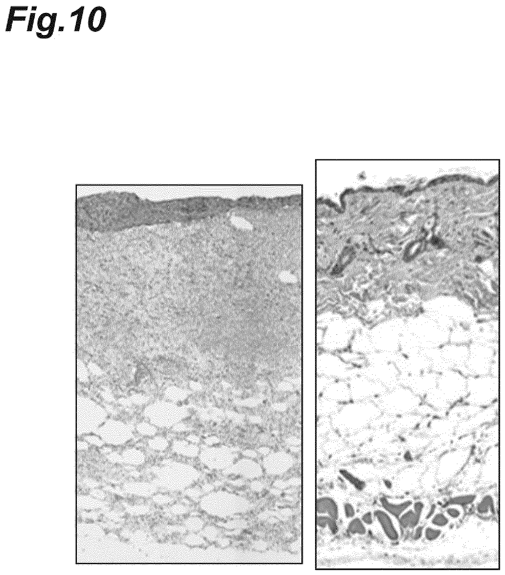

[Figure 10] Figure 10 is photographs showing the HE staining results of an artificial skin (left) in Example 3, which was produced, using $1 \times 10^6$ adipocytes and 10 mg fragmented collagen, at day 9 of culturing (keratinocyte day 7 of differentiation) and a mouse subcutaneous tissue-containing skin (right).

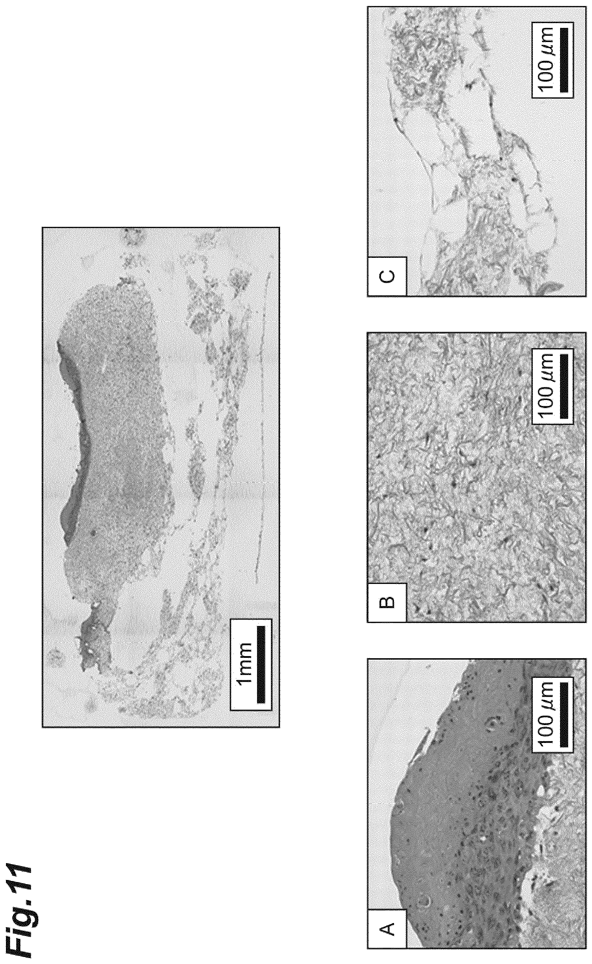

[Figure 11] Figure 11 is photographs showing the HE staining results of an artificial skin in Example 3, which was produced, using $1 \times 10^6$ adipocytes and 10 mg fragmented collagen, at day 9 of culturing (keratinocyte day 7 of differentiation). In photograph A, a keratinocyte-containing layer is enlarged. In photograph B, a fibroblast-containing layer is enlarged. In photograph C, an adipocyte-containing layer is enlarged.

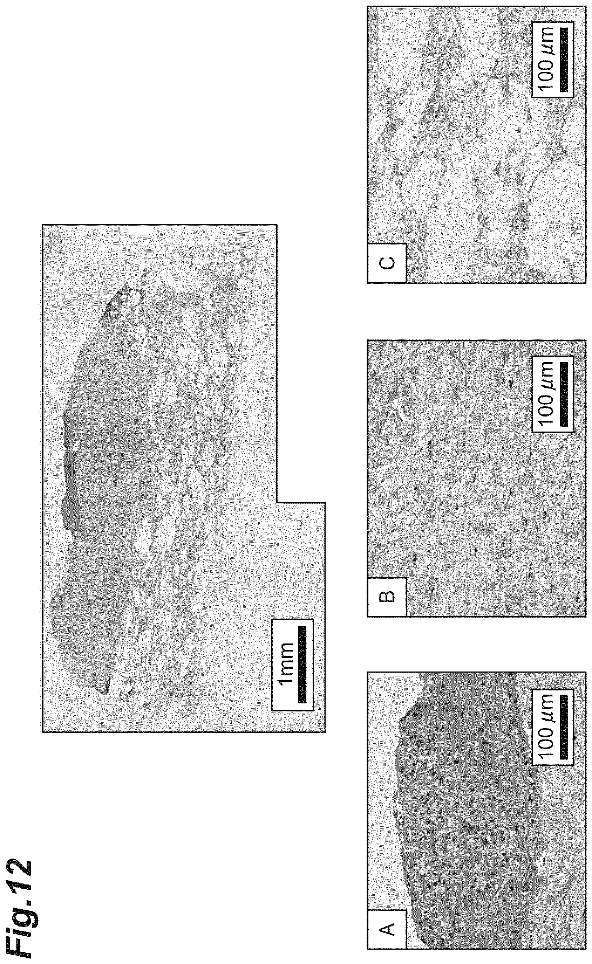

[Figure 12] Figure 12 is photographs showing the HE staining results of an artificial skin in Example 3, which was produced, using $5 \times 10^5$ adipocytes and 10 mg fragmented collagen, at day 9 of culturing (keratinocyte day 7 of differentiation). In photograph A, a keratinocyte-containing layer is enlarged. In photograph B, a fibroblast-containing layer is enlarged. In photograph C, an adipocyte-containing layer is enlarged.

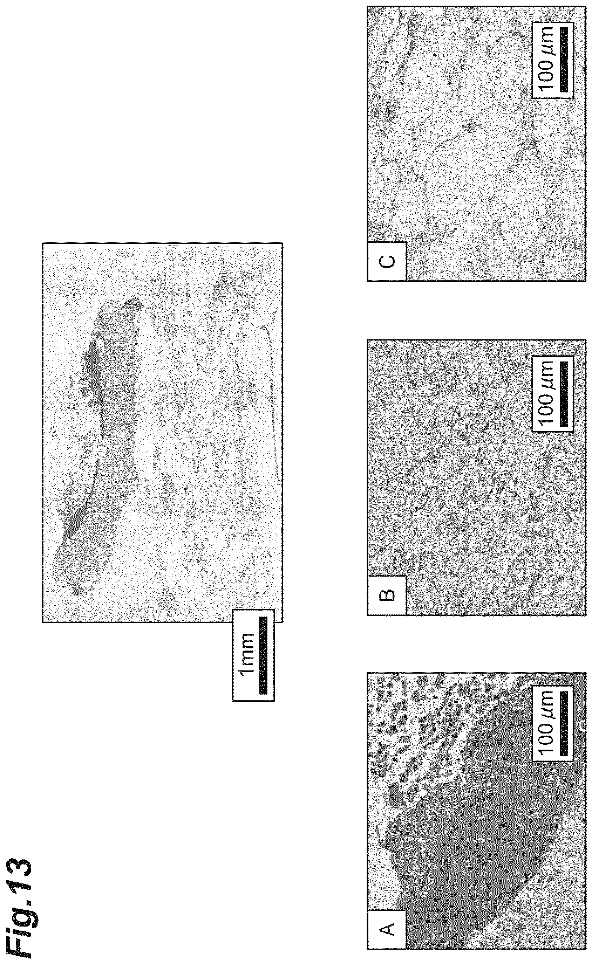

[Figure 13] Figure 13 is photographs showing the HE staining results of an artificial skin in Example 3, which was produced, using $1 \times 10^6$ adipocytes and 15 mg fragmented collagen, at day 9 of culturing (keratinocyte day 7 of differentiation). In photograph A, a keratinocyte-containing layer is enlarged. In photograph B, a fibroblast-containing layer is enlarged. In photograph C, an adipocyte-containing layer is enlarged.

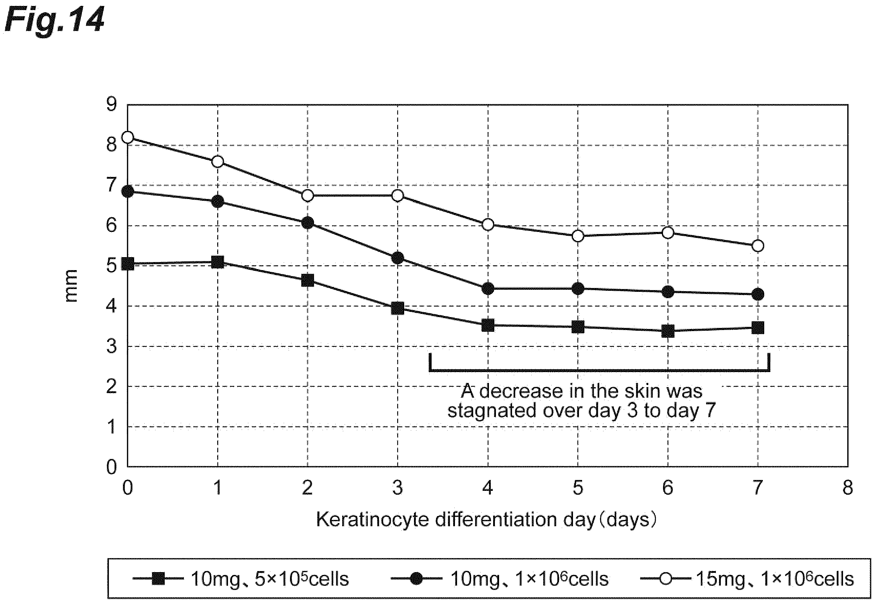

[Figure 14] Figure 14 is a graph showing the relationship between the keratinocyte differentiation time course and the artificial skin thicknesses of artificial skins having a three-layer structure produced in Example 3. The closed squares indicate the results of the artificial skin produced using $5 \times 10^5$ adipocytes and 10 mg fragmented collagen in Example 3. The closed circles indicate the results of the artificial skin produced using $1 \times 10^6$ adipocytes and 10 mg fragmented collagen in Example 3. The open circles indicate the results of the artificial skin produced using $1 \times 10^6$ adipocytes and 15 mg fragmented collagen in Example 3.

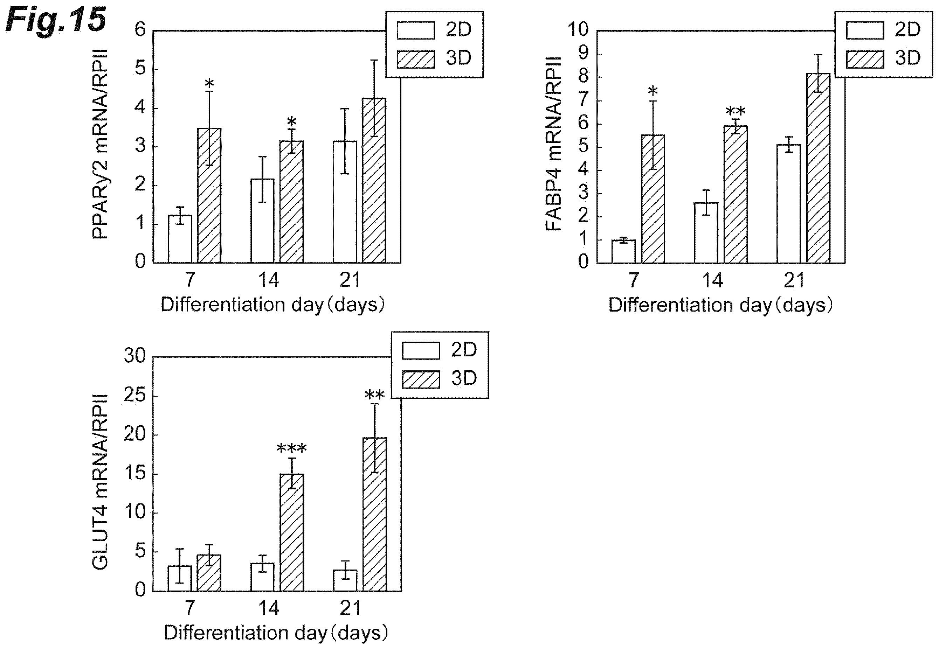

[Figure 15] Figure 15 shows the results of evaluating, using RT-qPCR, the levels of expression of adipogenesis genes *PPAR$\gamma$2, FABP4,* and *GLUT4* until day 21 of differentiation between a three-dimensional human adipose stem cell-containing tissue (3D) and an artificial adipose tissue (2D) produced by conventional two-dimensional culture, which levels are normalized by the level of expression of a housekeeping gene RPII. The "*" indicates a significant difference with respect to the two-dimensional tissue (*p < 0.05, **p < 0.01, and ***p < 0.001).

## Description of Embodiments

(Artificial Adipose Tissue)

An artificial adipose tissue according to an embodiment of the invention includes a three-dimensional tissue including cells containing an adipocyte and a fragmented extracellular matrix. At least part of the cells adhere to the fragmented extracellular matrix. Differentiation of the artificial adipose tissue according to this embodiment is promoted more than that of an artificial adipose tissue produced by conventional two-dimensional culture on a flat dish, and the artificial adipose tissue according to this embodiment can be maintained over a long period of time.

The artificial adipose tissue according to this embodiment is an artificially prepared adipose tissue and does not include an adipose tissue simply isolated from a biological tissue.

The "three-dimensional tissue" is a cell assembly in which cells are three-dimensionally arranged via an extracellular matrix such as collagen and means an assembly artificially prepared by cell culture. The shape of the three-dimensional tissue is not particularly limited. Examples include a sheet shape, a spherical shape, an ellipsoid shape, and a rectangular parallelepiped shape. Here, the artificial adipose tissue of this embodiment, which includes a three-dimensional tissue containing a fragmented extracellular matrix, is distinguishable, depending on the presence or absence of the fragmented extracellular matrix, from a biological tissue or a three-dimensional tissue produced by other methods without using the fragmented extracellular matrix.

Examples of the "adipocyte" in this embodiment include not only a differentiated cell such as a mature adipocyte but also an undifferentiated cell such as an adipose stem cell. As the adipocyte(s), it is possible to use a cell(s) collected from, for instance, a subcutaneous adipose tissue or an epicardium-derived adipose tissue. Also, differentiation of the collected cell(s) may be induced and then used. The adipocyte(s) is not particularly limited. It is preferable that when an adipose tissue constructed from adipocytes is utilized while finally seen as an *in vivo* tissue at a specific site, adipocytes derived from a tissue corresponding to the tissue at the site are used. Examples of animal species of the adipocyte include a human, a mouse, a rat, or a pig. Preferable examples of the adipocyte(s) include a mature adipocyte(s) or an adipose stem cell(s).

The "extracellular matrix" means an extracellularly existing material in an organism. Specific examples include collagen, elastin, proteoglycan, fibronectin, or hyaluronic acid. The extracellular matrix in this embodiment is preferably a material extracellularly present in an animal, namely, an extracellular matrix in an animal, more preferably contains collagen or elastin, is still more preferably collagen or elastin, and is particularly preferably collagen.

Examples of the collagen include fibrillar collagen or non-fibrillar collagen. The fibrillar collagen means collagen that is a main component of collagen fiber. Specific examples of the fibrillar collagen include type I collagen, type II collagen, or type III collagen. Examples of the non-fibrillar collagen include type IV collagen.

Conventional three-dimensional tissue bodies have had a low concentration of extracellular matrix such as collagen and a high cell density. Thus, there have been problems that the three-dimensional tissue bodies are contracted by cell contraction force during or after culturing and the three-dimensional tissue bodies are readily lysed by an enzyme(s) produced by cells during or after culturing. In addition, although porous high-density collagen, for instance, is commercially available, it cannot cause collagen and cells to evenly adhere and it is thus hard to recover cells to evaluate the cell characteristics. An adipose tissue including a three-dimensional tissue according to this embodiment has a higher extracellular matrix content than those of conventional three-dimensional tissue bodies. Accordingly, the adipose tissue is unlikely to contract and is thus stable.

The extracellular matrix content in the three-dimensional tissue may be from 0.01 to 90 wt%, preferably from 10 to 90 wt%, preferably from 1 to 50 wt%, more preferably from 10 to 30 wt%, and particularly preferably from 20 to 30 wt% based on the above three-dimensional tissue. As used herein, the "extracellular matrix in a three-dimensional tissue" means an extracellular matrix as a component of the three-dimensional tissue, and may be an endogenous or exogenous extracellular matrix. Also, examples of the "extracellular matrix in a three-dimensional tissue" include a fragmented extracellular matrix described later. Specifically, if the three-dimensional tissue includes an endogenous extracellular matrix, the concentration of extracellular matrix as a component of the above three-dimensional tissue means a sum of the concentrations of the endogenous extracellular matrix and the fragmented extracellular matrix. The above extracellular matrix concentration may be calculated from the volume of the resulting three-dimensional tissue and the mass of the decellularized three-dimensional tissue.

The "endogenous extracellular matrix" means an extracellular matrix produced by extracellular matrix-producing cells. The "endogenous collagen" means collagen produced by collagen-producing cells as a component of the three-dimensional tissue. The endogenous collagen may be fibrillar collagen and/or non-fibrillar collagen.

The "exogenous extracellular matrix" means an extracellular matrix that is externally supplied. An artificial adipose tissue according to this embodiment includes a three-dimensional tissue, which includes a fragmented extracellular matrix. An original animal species of the exogenous extracellular matrix may be the same as of exogenous extracellular matrix or may be different therefrom. Examples of the original animal species include a human, a pig, and a cow. In addition, the exogenous extracellular matrix may be a synthetic extracellular matrix. The fragmented extracellular matrix is an exogenous extracellular matrix. In the case of collagen, the extracellular matrix is called "exogenous collagen" and means collagen that is externally supplied. Specific examples include fibrillar collagen or non-fibrillar collagen. It is preferable that the exogenous collagen be fibrillar collagen. Examples of the fibrillar collagen include type I collagen, type II collagen, and type III collagen. Preferred is type I collagen. Commercially available collagen may be used as the above fibrillar collagen. Specific examples include lyophilized, pig skin-derived type I collagen manufactured by NH Foods Ltd. Examples of the non-fibrillar exogenous collagen include type IV collagen.

An original animal species of the exogenous extracellular matrix may be different from that of the cells. When the cells contain extracellular matrix-producing cells, an original animal species of the exogenous extracellular matrix may be different from that of the extracellular matrix-producing cells. That is, the exogenous extracellular matrix may be a heterologous extracellular matrix.

The above three-dimensional tissue includes a fragmented extracellular matrix. The "fragmented extracellular matrix" means those obtained by fragmenting an extracellular matrix such as collagen. Regarding an extracellular matrix as a source of the fragmented extracellular matrix, one kind may be used, or multiple kinds of extracellular matrix may be used in combination. Conventionally, an extracellular matrix such as collagen has been dissolved in, for instance, an acidic aqueous solution. However, the concentration is from about 0.1 to 0.3 wt%. Accordingly, a large amount of extracellular matrix cannot be dissolved. Consequently, it is difficult to increase the amount of extracellular matrix, such as collagen, in the three-dimensional tissue by conventional methods. A fragmented extracellular matrix in this embodiment is almost immiscible in water. However, when dispersed in the below-described aqueous medium, the fragmented extracellular matrix is likely to be contacted with cells, which include an adipocyte, in the aqueous medium, thereby possibly promoting formation of the three-dimensional tissue. The average length of the fragmented extracellular matrix is preferably from 100 nm to 200 $\mu$m, more preferably from 22 $\mu$m to 200 $\mu$m, and still more preferably from 100 $\mu$m to 200 $\mu$m. The average diameter of the fragmented extracellular matrix is preferably from 50 nm to 30 $\mu$m, more preferably

from 4 μm to 30 μm, and still more preferably from 20 μm to 30 μm.

When the extracellular matrix is collagen, the fragmented extracellular matrix is also called "fragmented collagen". The "fragmented collagen" is those produced by fragmenting collagen such as fibrillar collagen and means those in which a triple helix structure is kept. The average length of the fragmented collagen is preferably from 100 nm to 200 μm, more preferably from 22 μm to 200 μm, and still more preferably from 100 μm to 200 μm. The average diameter of the fragmented collagen is preferably from 50 nm to 30 μm, more preferably from 4 μm to 30 μm, and still more preferably from 20 μm to 30 μm.

A process for fragmenting an extracellular matrix such as collagen is not particularly limited. For instance, the extracellular matrix may be fragmented by using a homogenizer such as an ultrasonic homogenizer, a stirring homogenizer, or a high-pressure homogenizer. When the stirring homogenizer is used, an extracellular matrix, as it is, may be homogenized or may be homogenized in an aqueous medium such as saline. In addition, the duration and frequency of the homogenization may be adjusted to produce a fragmented extracellular matrix in mm or nm size.

The diameter and the length of the fragmented extracellular matrix may be calculated by analyzing individual fragmented extracellular matrix by electron microscopy.

A production method according to an embodiment of the invention makes it possible to produce an artificial adipose tissue, which includes a large-size three-dimensional tissue, with a thickness of 1 mm or more by using a relatively small number of cells.

The thickness of the artificial adipose tissue including the above three-dimensional tissue is preferably from 10 μm to 20 mm, more preferably from 100 μm to 15 mm, and particularly preferably from 1 mm to 10 mm. The lower limit of the thickness is not particularly limited and may be, for instance, 10 μm, 50 μm, 100 μm, 200 μm, 500 μm, 800 μm, 1 mm, 3 mm, or 5 mm. The upper limit of the thickness is not particularly limited and may be, for instance, 20 mm, 15 mm, 10 mm, 5 mm, 3 mm, 2 mm, 1.5 mm, or 1 mm. Such a three-dimensional tissue has a structure closer to the structure of a biological tissue, and is suitable as alternatives for experimental animals and graft materials.

As used herein, the "thickness of a three-dimensional tissue" means the distance between both ends in a direction vertical to the main surface when the three-dimensional tissue has a sheet shape or a rectangular parallelepiped shape. If the main surface has recess and/or a protrusion, the thickness means the distance across the thinnest portion of the main surface. When the three-dimensional tissue is spherical, the thickness means the diameter. Furthermore, when the three-dimensional tissue is elliptical, the thickness means the short diameter. If the three-dimensional tissue is substantially spherical or substantially elliptical with a recess and/or a protrusion on the surface, the thickness means the shortest distance between two points at which the surface intersects a line crossing the center of gravity of the three-dimensional tissue.

The artificial adipose tissue, which includes the three-dimensional tissue, has contraction rate during culturing of preferably 20% or less, more preferably 15% or less, and still more preferably 10% or less. The contraction rate may be calculated by, for instance, the following equation. In the equation, L1 denotes the length of the longest portion in the artificial adipose tissue including the three-dimensional tissue at day 1 of culturing; and L3 denotes the length of the corresponding portion in the artificial adipose tissue including the three-dimensional tissue at day 3 of culturing.

$$\text{Contraction rate } (\%) = [(L1 - L3)/L1] \times 100.$$

In the above example, the contraction rate is calculated from the artificial adipose tissue at day 1 of culturing and the artificial adipose tissue at day 3 of culturing. However, the contraction rate may be calculated from the artificial adipose tissue at day 1 of culturing and the artificial adipose tissue at day 2 of culturing. Also, the contraction rate may be calculated from the artificial adipose tissue at day 1 of culturing and the artificial adipose tissue at day 5 of culturing. In addition, the contraction rate may be calculated from the artificial adipose tissue at day 1 of culturing and the artificial adipose tissue at day 8 of culturing.

The size of lipid droplet(s) may be used as an indicator for indicating the maturity of each adipocyte cultured. The lipid droplet is an intracellular organelle in which lipids such as triglyceride (neutral fat) and cholesterol are stored and has a liquid droplet-like shape such that the above lipids are covered by a phospholipid monolayer. In addition, an adipose tissue-specific protein (e.g., Perilipin) is expressed on the surface of the above phospholipid. Lipid droplets in mature adipocytes vary in size. For instance, if the average lipid droplet size is 20 μm or more, the adipocyte is substantially matured and can thus be a mature adipocyte.

The average size of lipid droplets in adipocytes in the artificial adipose tissue of this embodiment is preferably from 20 μm to 180 μm and more preferably from 100 μm to 180 μm. The above lipid droplet size may be 250 μm or less, 200 μm or less, 180 μm or less, or 150 μm or less. Also, the above lipid droplet size may be 15 μm or more, 30 μm or more, 50 μm or more, or 80 μm or more. In addition, the above lipid droplet size may be a number at day 7 of culturing, day 10 of culturing, day 14 of culturing, or day 21 of culturing.

As an indicator for indicating the maturity of adipocytes cultured, it is possible to use the expression profile of gene markers such as an adipogenesis marker and a lipolysis marker. Examples of the adipogenesis marker include Peroxisome Proliferator-Activated Receptor $\gamma 2$ (PPAP$\gamma 2$), Fatty Acid Binding Protein 4 (FABP4), and Glucose transporter type 4 (GLUT-4). PPAR$\gamma 2$ and FABP4 can be utilized as early adipogenesis markers, and GLUT-4 can be utilized as a late adipogenesis marker. Examples of the lipolysis marker include hormone-sensitive lipase (HSL) and Adipocyte Triglyceride Lipase (ATGL).

PPAR$\gamma 2$ is one of the most important transcription factors during differentiation of adipocytes. During adipogenesis, the level of expression of PPAR$\gamma 2$ reportedly increases and is then constant or decreases (B. Galateanu et al., Int. J. Mol. Sci. 2012 (13) 15881-15900 and A. Soukas et al., J. Biol. Chem. 2001 (276) 34167-34174). Accordingly, it is considered that adipogenesis is advanced more in a tissue at the PPAR$\gamma 2$ constant expression stage than, for instance, in a tissue at the PPAR$\gamma 2$ increasing stage. FABP4 is another early marker and is needed for membrane transport so as to traffic fatty acids. Accordingly, a tissue exhibiting a more typical linearly increasing FABP4 expression profile, for example, is considered to be at an earlier adipogenesis stage. Regarding the typical linearly increasing FABP4 expression profile, E. C. M. Mariman et al., Cell. Mol. Life Sci. 2010 (67) 1277-1292 can be consulted.

Insulin-stimulated GLUT-4 is a major glucose transporter in adipocytes. The level of expression of GLUT-4 is weak in a tissue at the initial stage of differentiation during adipogenesis (S.-W. Qian et al., BMC Dev. Biol. 2010 (10) 47). Thus, the adipogenesis differentiation in a tissue having a more rapid increase in the level of expression of GLUT4, for instance, is considered to be advanced more.

The artificial adipose tissue of this embodiment may contain cells other than an adipocyte and may contain an extracellular matrix-producing cell. The "extracellular matrix-producing cell" means a cell that secretes an extracellular matrix such as collagen. That is, this artificial adipose tissue may contain an endogenously produced extracellular matrix. Examples of the extracellular matrix-producing cell include mesenchymal cells such as fibroblasts, chondrocytes, or osteoblasts. Preferred are fibroblasts. Examples of the preferable fibroblasts include human dermal fibroblasts (NHDF), human cardiac fibroblasts (NHCF), and human gingival fibroblasts (HGF). Examples of the cells other than the adipocyte include vascular endothelial cells (e.g., human umbilical vein endothelial cells (HUVECs)), cancer cells such as colon cancer cells (e.g., human colon cancer cells (HT29)) and liver cancer cells, cardiomyocytes (e.g., human iPS cell-derived cardiomyocytes (iPS-CMs)), epithelial cells (e.g., human gingival epithelial cells), lymphatic endothelial cells, neurons, hepatocytes, tissue stem cells, embryonic stem cells, induced pluripotent stem cells, adherent cells (e.g., immune cells), smooth muscle cells (e.g., aortic smooth muscle cells (Aorta-SMCs)), and keratinocytes (e.g., human epithelial keratinocytes).

The adipose tissue including the above three-dimensional tissue is applicable as alternatives for experimental animals, and graft materials etc. Specifically, the adipose tissue is applicable to, for instance, a cellulite, cosmetic assay screening for diabetes, etc., pharmaceutical screening for diabetes, etc., *in vitro* models for adipose tissue-related inflammatory disease or other pathologies, or tissue reconstruction after mastectomy or soft tissue lesion caused by trauma or tumorectomy.

(Artificial Skin)

An artificial skin according to an embodiment of the invention includes multiple layers, one of which is a layer including the above-described artificial adipose tissue. Specifically, the artificial skin according to this embodiment includes a first layer and a second layer, wherein the above-described artificial adipose tissue is included as the first layer. This artificial skin may include at least the first layer and the second layer, further optionally include a third layer, a fourth layer, and a fifth layer, and may also further include a layer(s). The order of the respective layers may be determined arbitrarily. However, the layers are preferably stacked in the order of from the first layer to the second layer to the third layer. In this case, as long as the first layer, the second layer, and the third layer are stacked in this order, for instance, another layer may be included, without restriction, under the first layer, between the first layer and the second layer, between the second layer and the third layer, or over the third layer.

The respective layers in the artificial skin according to this embodiment are distinguishable based on the kind of cells included and the difference in the cell distribution, etc. For instance, the artificial skin may be stained with different colors depending on the kinds of cells. Then, each layer is indicated with a different color, so that each layer is distinguishable in cross-section pictures, etc. Also, for instance, all the cells included in the artificial skin are stained. Then, each layer is distinguishable because there is a difference in the density of cells stained in the cross-section pictures, etc.

The layers (e.g., the second, third, fourth, and fifth layers) other than the first layer contain cells. The cells may include the above-described adipocyte and may include a cell other than the adipocyte. The second layer preferably contains fibroblasts or keratinocytes, more preferably contains fibroblasts, and still more preferably contains human dermal fibroblasts or human cardiac fibroblasts. The third layer preferably contains fibroblasts or keratinocytes, more preferably contains keratinocytes, and still more preferably contains human epithelial keratinocytes. When the first layer is the bottom layer, it is preferable that the second layer contains fibroblasts and the third layer contains keratinocytes.

When the layers are stacked in this way, an artificial skin closer to an *in vivo* human skin structure can be obtained.

In addition, the layers (e.g., the second, third, fourth, and fifth layers) other than the first layer may contain an extracellular matrix and may contain a fragmented extracellular matrix. The second layer preferably contains a fragmented extracellular matrix. Those described above may be used as the extracellular matrix and the fragmented extracellular matrix. Inclusion of the fragmented extracellular matrix allows for formation of a layer with suitable intercell distance.

The thickness of the artificial skin according to this embodiment is preferably from 10 μm to 20 mm, more preferably from 100 μm to 15 mm, still more preferably from 200 μm to 10 mm, and particularly preferably from 1 mm to 10 mm. The lower limit of the thickness is not particularly limited and may be, for instance, 10 μm, 50 μm, 100 μm 200 μm, 500 μm, 800 μm, 1 mm, 3 mm, or 5 mm. The upper limit of the thickness is not particularly limited and may be, for instance, 20 mm, 15 mm, 10 mm, 5 mm, 3 mm, 2 mm, 1.5 mm, or 1 mm. Such an artificial skin has a structure closer to the structure of a biological tissue, and is suitable as alternatives for experimental animals and graft materials.

The "thickness of artificial skin" means the distance from the tissue surface of the top layer to the tissue bottom surface of the bottom layer. For instance, in the case of a three-layer tissue including epithelial, dermal, and subcutaneous tissues, the thickness means the distance from the surface of the epithelial tissue to the bottom surface of the bottom layer, namely, the subcutaneous tissue (the total thickness of the three-layer tissue).

The number of cells included in the artificial skin according to this embodiment may be $1 \times 10^6$ or higher.

The first layer in the artificial skin of this embodiment includes the above-described artificial adipose tissue. The average size of lipid droplets in adipocytes in the artificial adipose tissue is preferably from 20 μm to 180 μm and more preferably from 100 μm to 180 μm. The above lipid droplet size may be 250 μm or less, 200 μm or less, 180 μm or less, or 150 μm or less. Also, the above lipid droplet size may be 15 μm or more, 30 μm or more, 50 μm or more, or 80 μm or more. In addition, the above lipid droplet size may be a number at day 7 of culturing, day 10 of culturing, day 14 of culturing, or day 21 of culturing.

The above artificial skin may be used as an alternative for *in vivo* skin and is applicable as an alternatives for experimental animals, graft materials, etc. Specifically, the artificial skin is applicable to, for instance, tests for evaluating skin permeability, skin safety, skin corrosion, skin irritancy, or the like of a compound, cosmetic assay screening, pharmaceutical screening, *in vitro* pathological models, and/or graft materials for plastic surgery or burn operation.

(Method for Producing Artificial Adipose Tissue)

A method for producing an artificial adipose tissue according to an embodiment of the invention includes:

(1) contacting cells containing an adipocyte with a fragmented extracellular matrix in an aqueous medium (hereinafter, sometimes referred to as step (1)); and
(2) culturing the cells contacted with the fragmented extracellular matrix (hereinafter, sometimes referred to as step (2)).

The "adipocyte" and the "fragmented extracellular matrix", etc., are as described above.

The "cells containing an adipocyte" in step (1) of this embodiment may contain cells other than an adipocyte and may contain an extracellular matrix-producing cell. The "extracellular matrix-producing cell" means a cell that secretes an extracellular matrix such as collagen. Examples of the extracellular matrix-producing cell include mesenchymal cells such as fibroblasts, chondrocytes, or osteoblasts. Preferred are fibroblasts. Examples of the preferable fibroblasts include human dermal fibroblasts (NHDF), human cardiac fibroblasts (NHCF), and human gingival fibroblasts (HGF). Examples of the cells other than the adipocyte include vascular endothelial cells (e.g., human umbilical vein endothelial cells (HUVECs)), cancer cells such as colon cancer cells (e.g., human colon cancer cells (HT29)) and liver cancer cells, cardiomyocytes (e.g., human iPS cell-derived cardiomyocytes (iPS-CMs)), epithelial cells (e.g., human gingival epithelial cells), lymphatic endothelial cells, neurons, hepatocytes, tissue stem cells, embryonic stem cells, induced pluripotent stem cells, adherent cells (e.g., immune cells), smooth muscle cells (e.g., aortic smooth muscle cells (Aorta-SMCs)), and keratinocytes (e.g., human epithelial keratinocytes).

The "aqueous medium" means liquid using water as an essential component. As long as the fragmented collagen and the cells are present stably, the aqueous medium has no particular restriction. Examples include saline such as phosphate buffered saline (PBS) and liquid culture medium such as Dulbecco's Modified Eagle culture medium (DMEM), or vascular endothelial cell-specific culture medium (EGM2). A mixed culture medium, in which two different culture media are mixed, is acceptable as the liquid culture medium. From the viewpoint of decreasing a load against cells, it is preferable that the aqueous medium is liquid culture medium.

A procedure for contacting cells containing an adipocyte with a fragmented extracellular matrix in an aqueous medium is not particularly limited. Examples include: a procedure for adding a fragmented extracellular matrix-containing dispersion to a culture liquid containing cells; a procedure for adding cells to a fragmented extracellular matrix-containing culture medium dispersion; or a procedure for separately adding a fragmented extracellular matrix and cells to a beforehand prepared aqueous medium.

The concentration of the fragmented extracellular matrix in the aqueous medium in step (1) may be determined, if appropriate, depending on the shape and the thickness of artificial adipose tissue including the three-dimensional tissue of interest, the size of cultureware, and so on. For instance, the concentration of the fragmented extracellular matrix in the aqueous medium in step (1) may be from 0.1 to 90 wt% and may be from 1 to 30 wt%.

The amount of the fragmented extracellular matrix in step (1) may be from 0.1 to 100 mg and may be from 1 to 50 mg with respect to $1 \times 10^5$ cells.

The mass ratio between the fragmented extracellular matrix and the cells in step (1) is preferably from 1000 : 1 to 1 : 1, more preferably from 900 : 1 to 9 : 1, and still more preferably from 500 : 1 to 10 : 1.

When the adipocyte and the other cells are together used, the (cell count) ratio between the adipocyte and the other cells in step (1) may be from 99 : 1 to 9 : 1 and may be from 80 : 20 to 50 : 50.

The production method may further include, between step (1) and step (2), the step of subjecting the cells containing the adipocyte and the fragmented extracellular matrix in the aqueous medium to precipitate. Such a step may be conducted to produce a more uniform distribution of the cells and the fragmented extracellular matrix in the artificial adipose tissue including the three-dimensional tissue. The specific procedure is not particularly limited. Examples include a procedure in which a culture liquid containing the fragmented extracellular matrix and the cells is centrifuged.

In step (1), the step may be implemented by forming a cell layer in the aqueous medium and then by contacting it with the fragmented extracellular matrix. The cell layer may be formed before the contact with the fragmented extracellular matrix to produce an artificial adipose tissue including a three-dimensional tissue, a lower layer of which has a high cell density. For instance, the cell layer containing an extracellular matrix-producing cell is formed before the contact with the fragmented extracellular matrix. This makes it possible to produce an artificial adipose tissue including a three-dimensional tissue having a high cell density in the lower layer portion including the cells containing an extracellular matrix-producing cell. Depending on the kinds of cells used, this method allows for production of an artificial adipose tissue closer to *in vivo* one.

In this embodiment, the method for producing a three-dimensional tissue optionally includes, as step (3) after step (2), the step of further contacting it with cells and culturing the cells. These cells may be the same as or different from the cells used in step (1). For instance, when the cells used in step (1) contain cells other than an extracellular matrix-producing cell, the cells used in step (3) may contain an extracellular matrix-producing cell. Also, for instance, when the cells used in step (1) contain an extracellular matrix-producing cell, the cells used in step (3) may contain cells other than an extracellular matrix-producing cell. Both the cells used in step (1) and the cells used in step (3) may contain an extracellular matrix-producing cell. Both the cells used in step (1) and the cells used in step (3) may contain cells other than an extracellular matrix-producing cell. The above step (3) allows for production of an artificial adipose tissue including a three-dimensional tissue with a double layer structure. Substantially the same method may be used to produce an artificial skin including multiple layers. A method for producing this artificial skin will be described below.

In step (2), a procedure for culturing the cells contacted with the fragmented extracellular matrix is not particularly limited and may be implemented using a suitable culture protocol depending on the kind of cells cultured. For instance, the culturing temperature may be from 20°C to 40°C and may be from 30°C to 37°C. The pH of the culture medium may be from 6 to 8 and may be from 7.2 to 7.4. The production method according to this embodiment does not require a culture medium with a complicated composition as used for conventional three-dimensional culture or three-dimensional tissue production. A culture medium, such as DMEM, that can be prepared easily may be used. The culture medium is not particularly limited, and a suitable culture medium can be selected depending on the kinds of cells cultured. Examples of the culture medium include Eagle's MEM culture medium, DMEM, Modified Eagle Medium (MEM), Minimum Essential culture medium, RPMI, or GlutaMax culture medium. The culture medium may be serum-containing medium or serum-free medium. A mixed culture medium, in which two different culture media are mixed, is acceptable as the culture medium. In addition, the production method according to this embodiment makes it possible to produce a fully differentiated artificial adipose tissue in a much shorter culturing period than that of conventional two-dimensional culture. Thus, the culturing period in step (2) may be, for instance, from 10 days to 60 days, from 10 days to 30 days, from 13 days to 25 days, or from 14 days to 17 days. Provided that the above culturing period just represents a period required for obtaining a fully differentiated adipose tissue and is not set to prohibit culturing of the period or longer.

The cell density in the culture medium in step (2) may be determined, if appropriate, depending on the shape and the thickness of three-dimensional tissue of interest, the size of cultureware, and so on. For instance, the cell density in the culture medium in step (2) may be from 1 to $10^8$ cells/mL and may be from $10^3$ to $10^7$ cells/mL. In addition, the cell density in the culture medium in step (2) may be the same as that in the aqueous medium in step (1).

The artificial adipose tissue, which includes the three-dimensional tissue produced by the production method according to this embodiment, has the contraction rate during culturing of preferably 20% or less, more preferably 15% or less, and still more preferably 10% or less. The contraction rate may be calculated by, for instance, the following equation. In the equation, L1 denotes the length of the longest portion in the three-dimensional tissue at day 1 of culturing; and L3 denotes the length of the corresponding portion in the three-dimensional tissue at day 3 of culturing.

$$\text{Contraction rate (\%)} = [(L1 - L3)/L1] \times 100.$$

In the above example, the contraction rate is calculated from the artificial adipose tissue at day 1 of culturing and the artificial adipose tissue at day 3 of culturing. The contraction rate may be calculated from the artificial adipose tissues at any culturing time points including the end point of culturing. For instance, the contraction rate may be calculated from the artificial adipose tissue at day 1 of culturing and the artificial adipose tissue at day 2 of culturing. Also, the contraction rate may be calculated from the artificial adipose tissue at day 1 of culturing and the artificial adipose tissue at day 5 of culturing. In addition, the contraction rate may be calculated from the artificial adipose tissue at day 1 of culturing and the artificial adipose tissue at day 8 of culturing.

The residual percentage of the artificial adipose tissue, which includes the three-dimensional tissue produced by the production method according to this embodiment, after trypsin treatment at a trypsin concentration of 0.25%, a temperature of 37°C, a pH of 7.4, and a reaction time of 15 min is preferably 70% or higher, more preferably 80% or higher, and still more preferably 90% or higher. Such a three-dimensional tissue is not susceptible to enzymatic degradation during or after culturing and is thus stable. The above residual percentage may be calculated, for instance, from the masses of the three-dimensional tissue before and after the trypsin treatment.

The residual percentage of the artificial adipose tissue, which includes the three-dimensional tissue produced by the production method according to this embodiment, after collagenase treatment at a collagenase concentration of 0.25%, a temperature of 37°C, a pH of 7.4, and a reaction time of 15 min is preferably 70% or higher, more preferably 80% or higher, and still more preferably 90% or higher. Such a three-dimensional tissue is not susceptible to enzymatic degradation during or after culturing and is thus stable.

In addition, the production method according to this embodiment allows for production of an artificial adipose tissue including a stable three-dimensional tissue in which cells are evenly distributed. Further, the artificial adipose tissue produced by the production method according to this embodiment can be maintained for a longer period than that produced by conventional two-dimensional culture. Furthermore, the production method according to this embodiment allows for production of an artificial adipose tissue including a large-size three-dimensional tissue having a relatively small number of cells and a thickness of 1 mm or more.

(Method for Producing Artificial Skin)

A method for producing an artificial skin according to an embodiment of the invention includes:

forming a first layer, including

(1) contacting first cells containing an adipocyte with a first fragmented extracellular matrix in an aqueous medium (hereinafter, referred to as step (1)'), and
(2) culturing the first cells contacted with the first fragmented extracellular matrix, thereby (hereinafter, sometimes referred to as step (2)'); and

forming a second layer, including (3) further contacting the first layer with second cells and culturing the second cells(hereinafter, sometimes referred to as step (3)').

Substantially the same procedures as describe above (for the artificial adipose tissue production method) are implemented in steps (1)' and (2)'. The aqueous medium, the first cells containing an adipocyte, the second cells, and the first fragmented extracellular matrix may be represented likewise by the above-described aqueous medium, the cells containing an adipocyte, and the fragmented extracellular matrix.

The procedure for forming the first layer in step (2)' is not particularly limited. As long as the thickness of cells cultured is a desired thickness, the layer may be considered to be formed. Step (3)' is not necessarily started after completion of the first layer formation in step (2)'. It is possible to start step (3)' during the culturing in step (2)'.

The average length of the first fragmented extracellular matrix is preferably from 100 nm to 200 $\mu$m, more preferably from 22 $\mu$m to 200 $\mu$m, and still more preferably from 100 $\mu$m to 200 $\mu$m. The average diameter of the fragmented extracellular matrix is preferably from 50 nm to 30 $\mu$m, more preferably from 4 $\mu$m to 30 $\mu$m, and still more preferably from 20 $\mu$m to 30 $\mu$m.

In addition, the production method according to this embodiment makes it possible to produce a fully differentiated adipose tissue (the first layer) in a much shorter culturing period than that of conventional two-dimensional culture. Thus, the culturing period in step (2)' may be, for instance, from 10 days to 60 days, from 10 days to 30 days, from 13 days to 25 days, or from 14 days to 17 days. Provided that the above culturing period just represents a period required for obtaining a fully differentiated adipose tissue and is not set to prohibit culturing of the period or longer.

The second cells may be the same as or different from the first cells. Substantially the same procedures as describe above (for the artificial adipose tissue production method) are implemented in the cell contact procedure and the culturing protocol.

Examples of the cells used as the second cells include substantially the same cells as mentioned above (for the artificial adipose tissue). Here, it is preferable that the second cells contain fibroblasts. When the second layer is a layer containing differentiated fibroblasts, the structure can be closer to that of *in vivo* skin.

In addition, step (3)' is preferably a step comprising contacting the second cells with a second fragmented extracellular matrix on the first layer in an aqueous medium and culturing the second cells. Examples of the second extracellular matrix include substantially the same one as mentioned above (for the artificial adipose tissue). Inclusion of the fragmented extracellular matrix allows for formation of a layer with suitable intercell distance. It is preferable that the second extracellular matrix contains collagen. It is more preferable that the second extracellular matrix is collagen.

The artificial skin production method according to this embodiment may further comprise forming a third layer including further contacting the second layer with third cells and culturing the third cells.

Examples of the cells used as the third cells include substantially the same cells as mentioned above (for the artificial adipose tissue). It is preferable that the third cells contain keratinocytes. When the third layer is a layer containing differentiated keratinocytes, the structure can be closer to that of *in vivo* skin.

As likewise described above (for the artificial skin), the artificial skin production method according to this embodiment makes it possible to produce an artificial skin with a thickness close to that of *in vivo* skin in a much shorter culturing period than that of conventional two-dimensional culture.

In addition, the artificial skin production method according to this embodiment allows for production of an artificial skin including, as the first layer, an artificial adipose tissue including a stable three-dimensional tissue in which cells are evenly distributed. Further, in the artificial adipose tissue in the artificial skin produced by the production method according to this embodiment, mature adipocytes can be maintained for a longer period than those during conventional two-dimensional culture. Furthermore, the production method according to this embodiment makes it possible to produce an artificial skin having a relatively small number of cells and a thickness of 1mm or longer, which thickness is close to that of *in vivo* skin.

<Agent for Adipocyte Culture>

An agent for adipocyte culture according to an embodiment of the invention contains a fragmented extracellular matrix. In the agent for adipocyte culture according to this embodiment, the average length of the fragmented extracellular matrix may be from 100 nm to 200 $\mu$m. The average diameter of the fragmented extracellular matrix may be from 50 nm to 30 $\mu$m. Here, the length of 95 % of the fragmented extracellular matrix with respect to the whole extracellular matrix may be ranged from 100 nm to 200 $\mu$m. Here, the diameter of 95 % of the fragmented extracellular matrix with respect to the whole extracellular matrix may be ranged from 50 nm to 30 $\mu$m.

The "agent for adipocyte culture" means a reagent for culturing adipocytes. The agent for adipocyte culture may be in a powder state or may be in a dispersion state in which the fragmented extracellular matrix is dispersed in an aqueous medium. Examples of the kind, size, and production method of the fragmented extracellular matrix and usage of the above culture agent include substantially the same ones as designated above (for the artificial adipose tissue) and (for the artificial adipose tissue production method). Inclusion of the fragmented extracellular matrix can promote differentiation of adipocytes. Thus, the agent for adipocyte culture may be understood as a differentiation promoter. In addition, inclusion of the fragmented extracellular matrix is unlikely to cause mature adipocytes to be dedifferentiated. Thus, the agent for adipocyte culture may also be understood as a mature adipocyte dedifferentiation inhibitor (agent for maintaining mature adipocytes). Further, the culture agent according to this embodiment makes it possible to form a tissue having less tissue contraction due to long-term culturing even when a tissue having a thickness of, for instance, 1 mm or more is formed. The culture agent can exert a differentiation-promoting effect and a dedifferentiation inhibitory effect to form a tissue, the shape of which is stable for a long period of time.

(Method for Evaluating Skin Permeability of Compound)

A method for evaluating skin permeability of a compound according to an embodiment of the invention comprises:

providing an artificial skin;
contacting the artificial skin with a test compound;
measuring skin permeability of the test compound using the artificial skin contacted with the test compound; and
comparing between the skin permeability of the test compound and a reference value.

As the artificial skin, it is possible to use an artificial skin according to the embodiment as described above (for the artificial skin) or an artificial skin produced by the production method according to the embodiment as described above (for the artificial skin production method). As stated above, this artificial skin has a structure and a thickness similar to those of *in vivo* skin and may be used, as an *in vivo* skin alternative, for a skin permeability test of a compound.

The test compound is not particularly limited and any test compound can be used as long as the compound is used in a regular skin permeability test.

A contact procedure for contacting the artificial skin with the test compound is not particularly limited. In the case of the test compound being liquid, the test compound may be, for example, directly applied or sprayed on the surface of the artificial skin. In the case of the test compound being solid, a solution in which the test compound has been dissolved in a solvent may be used to likewise apply or spray on the surface of the artificial skin.

The skin permeability of the test compound may be measured based on a skin permeability test protocol well-known to those skilled in the art. For instance, safety of a cosmetic/quasi drug of interest may be evaluated and tested in accordance with the guidance in Notification No. 1115-1 of the Central Pharmaceutical Affairs Council (November 15, 2016).

The reference value may be set, if appropriate, based on the kind and the concentration of the test compound. For instance, when the reference value is one point and the skin permeability is higher than the reference value, the skin permeability can be determined as high. When the skin permeability is lower than the reference value, the skin permeability can be determined as low. In addition, the reference value may be set as, for instance, a range, within which the skin permeability is ranked.

Hereinbelow, the invention will be specifically described in detail with reference to Examples. However, the Examples do not restrict the scope of the invention.


**Examples**

(Example 1: Production of Artificial Adipose Tissue Using Mouse Mature Adipocytes)

The present inventors developed a method for producing a three-dimensional tissue using collagen microfiber so as to increase collagen density in the tissue (Figure 1). An artificial adipose tissue using mature adipocytes was produced as illustrated below in the schematic diagram of Figure 1.

Lyophilized, pig skin-derived type I collagen manufactured by NH Foods Ltd. was dispersed in 10 × conc. phosphate buffered saline (×10 PBS) and homogenized using a homogenizer for 5 min to yield fragmented collagen with a diameter of about 4.4 $\mu$m and a length of about 22.5 $\mu$m. The resulting fragmented collagen was washed with serum-free culture medium (DMEM) to prepare a fragmented collagen-containing culture medium dispersion.

The fragmented collagen was dispersed at a concentration of 10 mg/ml in serum-containing culture medium (DMEM). Then, 1 mL of the resulting dispersion (corresponding to about 10 mg fragmented collagen) and $1 \times 10^6$ primary mature mouse adipocytes in DMEM were mixed in a 24-well plate transwell (manufactured by IWAKI, Inc.). Twenty-four hours after the seeding, the transwell was placed in a 6-well plate (manufactured by IWAKI, Inc.) containing 6 mL of culture medium (DMEM culture medium, manufactured by NACALAI TESQUE, INC.). The mature adipocytes were cultured until day 14 while the culture medium was changed every 4 days. After the start of culturing, the mixture containing the cells and the fragmented collagen was just subject to precipitate. Because of no centrifugation, the thickness decreased, over the culturing process, in the gravity direction and became stable to produce a three-dimensional tissue with a thickness of about 2 mm after 14 days of culturing. An artificial adipose tissue including the resulting three-dimensional tissue was subjected to hematoxylin/eosin (HE) staining and/or immunostaining with an anti-perilipin antibody. Then, histological evaluation was conducted. In addition, Nile red was used to stain the lipids. The viability was assessed using a Live/Dead kit (manufactured by Thermo Fisher Scientific, Inc.). The fat vesicles were analyzed and the Live/Dead was measured by ImageJ software (provided by National Institutes of Health in the U.S.). The fat vesicle diameters of 100 fat vesicles were measured by electron microscopy at day 7 of culturing and day 14 of culturing, and were compared to those of adipocytes in an artificial adipose tissue produced by conventional two-dimensional culture.

The method for producing an artificial adipose tissue by conventional two-dimensional culture was carried out as follows. First, $3 \times 10^5$ (cells/well) mouse primary mature adipocytes were seeded on a 6-well plate (manufactured by IWAKI, Inc.) and then cultured. The culturing was continued in 2 mL of culture medium (DMEM culture medium, manufactured by NACALAI TESQUE, INC.). The culture medium was changed every 3 days. Note that mature adipocytes do not adhere to the bottom surface of dish because lipid droplets cause floatation effects. Thus, a commercially available cover glass was floated on the culture medium, and the cells was adhered underneath during the culturing.

The HE staining revealed the presence of high-density fragmented collagen in the three-dimensional tissue surrounding the mature adipocytes (the arrow in Figure 2A). The mature adipocytes each had a small cytoplasm and a nucleus, and a typical round uniocular shape (Figure 2A).

The Nile red lipid staining demonstrated a lipid droplet(s) in the cytoplasm. A uniocular lipid droplet, which indicates a mature adipocyte, was found, indicating a homogenous, high redifferentiation of mature adipocytes in the three-dimensional tissue (Figure 2B). Even in the case of long-term culturing of 14 days or longer, the mature adipocytes in the artificial adipose tissue were well maintained at high viability of from 98.5% at day 2 to 94.6% at day 14 (in Figure 2C, gray indicates living cells and white indicates dead cells). The results of immunostaining with an anti-perilipin antibody demonstrated that the mature adipocytes in the three-dimensional tissue at day 14 of culturing maintained their larger uniocular shape (a fat vesicle with a size of 54 $\pm$ 8 $\mu$m at day 0) than adipocytes in an artificial adipose tissue produced by conventional two-dimensional culture (Figure 3). By contrast, the mature adipocytes in the artificial adipose tissue produced by conventional two-dimensional culture were dedifferentiated to a fibroblast-like cell morphology and had vesicles 3.7 times significantly smaller than those of the mature adipocytes in the three-dimensional tissue at day 14 of culturing (in Figure 4, n > 100 fat vesicles were counted; t-test). Also, in the artificial adipose tissue including the three-dimensional tissue containing fragmented collagen, the size of fat vesicles was maintained from day 7 to day 14. However, in the artificial adipose tissue produced by conventional two-dimensional culture, the size of fat vesicles decreased from day 7 to day 14. This indicates that the time to dedifferentiation was longer in the three-dimensional tissue containing fragmented collagen than in the artificial adipose tissue produced by conventional two-dimensional culture, so that the long-term maintenance was possible and the culturing period of the mature adipocytes was successfully extended by at least 1 week or longer.

(Example 2: Production of Artificial Adipose Tissue Using Human Adipose Stem Cells)

An artificial adipose tissue using adipose stem cells was produced as illustrated below in the schematic diagram of Figure 1.

Lyophilized, pig skin-derived type I collagen manufactured by NH Foods Ltd. was dispersed in 10 $\times$ conc. phosphate buffered saline ($\times$10 PBS) and homogenized using a homogenizer for 5 min to yield fragmented collagen with a diameter of about 4.4 $\mu$m and a length of about 22.5 $\mu$m. The resulting fragmented collagen was washed with serum-free medium (DMEM) to prepare a fragmented collagen-containing culture medium dispersion.

The fragmented collagen was dispersed at a concentration of 10 mg/ml in serum-containing culture medium (DMEM). Then, 300 $\mu$L of the resulting dispersion (corresponding to about 3 mg fragmented collagen) and $5 \times 10^5$ human adipose stem cells in DMEM were mixed in a 96-well plate insert (manufactured by ACEA Bioscience, Inc.). Twenty-four hours after the seeding, 200 $\mu$L of culture medium (DMEM culture medium, manufactured by NACALAI TESQUE, INC.) was added. The culture medium was changed every 2 days and the culturing was conducted for 19 days. Of 19 days, 2 days correspond to a growth period and the subsequent 17 days correspond to a differentiation period. After the start of culturing, the mixture containing the cells and the fragmented collagen was just subject to precipitate. Because of no centrifugation, the thickness decreased, over the culturing process, in the gravity direction and became stable to produce a three-dimensional tissue with a thickness of about 2 mm after 14 days of culturing. An artificial adipose tissue including the three-dimensional tissue at day 17 of differentiation (day 19 of culturing) was subjected to hematoxylin/eosin (HE) staining and/or immunostaining with an anti-perilipin antibody. Then, histological evaluation was conducted.

The HE staining revealed the presence of high-density fragmented collagen in the three-dimensional tissue (the arrow in Figure 5). A large round uniocular lipid droplet, which indicates a mature adipocyte, was found (a magnified view in Figure 5). Among them, large mature adipocytes with a size of more than 100 $\mu$m were also found.

A method for producing an artificial adipose tissue by conventional two-dimensional culture was carried out as follows. First, $1 \times 10^4$ (cells/well) human adipose stem cells were seeded on a 24-well plate (manufactured by IWAKI, Inc.) and then cultured. During 2 days after the seeding, 500 $\mu$L of D-MEM was used for the culturing. After their growth, the culture medium was changed to a differentiation-promoting culture medium. The differentiation-promoting culture medium was prepared by adding PGM-2 Singlequot Kit PT-9502 (manufactured by LONZA) to D-MEM and contained growth aids (Indomethacin, 3-isobutyl-1-methyl xanthine, dexamethasone, and insulin). The culturing was conducted in 500 $\mu$L, and the culture medium was changed once a week.

In the artificial adipose tissue produced by conventional two-dimensional culture, fat vesicles in a liquid droplet state were found here and there even at a time point of day 37 of differentiation (Figure 6). In addition, when examined by Nile red lipid staining, only small lipid droplets were found. Mature adipocytes with a size of more than 100 $\mu$m, like in the three-dimensional tissue containing fragmented collagen, were not found.

Further, in the artificial adipose tissue produced by conventional two-dimensional culture, fat vesicles in a liquid droplet state were found here and there even at a time point of day 72 of differentiation; and any of the adipocytes were not differentiated (Figure 6). Furthermore, as the fat content increased, the adipocytes detached and floated more in the culture liquid. As a result, it was difficult to change the culture liquid.

The results of immunostaining with an anti-perilipin antibody demonstrated that a larger uniocular shape (fat vesicle with a size of 4 $\mu$m $\pm$ 2 $\mu$m at day 7) was formed in the adipose stem cells in the three-dimensional tissue at day 7 of culturing than in adipose stem cells in an artificial adipose tissue produced by conventional two-dimensional culture (Figure 7). Then, the artificial adipose tissue produced by conventional two-dimensional culture had vesicles 2 times significantly smaller than those of the adipocytes in the three-dimensional tissue at day 7 of culturing (in Figure 8, n > 100 fat vesicles were counted; t-test). Here, in the artificial adipose tissue including the three-dimensional tissue containing fragmented collagen, the size of fat vesicles was doubled or more (10 $\mu$m $\pm$ 4 $\mu$m) from day 7 to day 14. However, in the artificial adipose tissue produced by conventional two-dimensional culture, the size of fat vesicles had almost no change from day 7 to day 14.

The above has demonstrated that differentiation of the artificial adipose tissue including the three-dimensional tissue containing fragmented collagen progresses more rapidly than that of the artificial adipose tissue produced by conventional two-dimensional culture.

(Example 3: Production of Artificial Skin Using Mature Adipocytes)

An artificial skin using mature adipocytes was produced as illustrated below in the schematic diagram of Figure 9.

Lyophilized, pig skin-derived type I collagen manufactured by NH Foods Ltd. was dispersed in 10 $\times$ conc. phosphate buffered saline ($\times$10 PBS) and homogenized using a homogenizer for 5 min to yield fragmented collagen with a diameter of about 4.4 $\mu$m and a length of about 22.5 $\mu$m. The resulting fragmented collagen was washed with serum-free medium (DMEM) to prepare a fragmented collagen-containing culture medium dispersion.

The fragmented collagen was dispersed at a concentration of 10 mg/ml in serum-containing culture medium (DMEM). Then, 1 mL of the resulting dispersion (corresponding to about 10 mg fragmented collagen) and 1 $\times$ 10$^6$ rat mature adipocytes, which had been collected from a rat subcutaneous tissue, in DMEM were mixed in a 24-well plate transwell (manufactured by IWAKI, Inc.). This plate was placed in an incubator, and the culturing was conducted for 24 h.

Next, a 24-well insert was coated with a 0.04 mg/mL fibronectin solution (#F2006-5G, manufactured by Sigma, Inc.) in PBS (0.04 $\mu$L/insert), and was incubated at 37°C for 20 min. Then, 1 mL of the above fragmented collagen-containing culture medium dispersion (corresponding to about 10 mg of fragmented collagen) and 5 $\times$ 10$^5$ human dermal fibroblasts (NHDF) were mixed, and 100 $\mu$L of the mixture was aliquoted in the 24-well insert. The plate was placed in an incubator, and the culturing was conducted for 24 h.

Next, the culture medium over the NHDF-containing collagen gel in the insert was aspirated. Then, the gel was coated with 0.04 mg/mL collagen IV solution in PBS (0.04 $\mu$L/insert) and was incubated at 37°C for at least 20 min. The collagen IV solution added onto the NHDF-containing collagen gel was aspirated. After that, 2 $\times$ 10$^6$ normal human epithelial keratinocytes (#KK-4009, 1 vial = 500000 cells, manufactured by KURABO, Inc.) were added onto the NHDF-containing collagen gel. Subsequently, 1 mL of DMEM 5% FBS : EpiLife (#C-2517A, manufactured by Invitrogen, Inc.) (1:1) culture medium was added to the outside of the insert. After 1 h, another 1 mL was added to the outside of the insert.

The culture media on the inside and the outside were gently aspirated. Next, ascorbic acid was diluted 100-fold in DMEM 5% FBS : EpiLife (1:1) culture medium, which was then added, as 500 $\mu$L of differentiation medium, to the outside of the insert. No culture medium was added to the inside of the insert. The culture medium outside the insert was changed every day until differentiation day 7.

The above method required just 9 days from the start of adipose tissue culturing (24 h of adipose tissue culturing + 24 h of fibroblast culturing + 7 days of keratinocyte differentiation) so as to be able to produce an artificial skin with a three-layer structure including a bottom adipose tissue layer, an intermediate fibroblast layer, and a top keratinocyte layer (Figure 9).

The artificial skin as so produced at keratinocyte differentiation day 7 was subjected to hematoxylin/eosin (HE) staining for histological evaluation. The above artificial skin had an appropriate intercell distance in any of the layers including the bottom adipocyte-containing layer (subcutaneous tissue), the intermediate fibroblast-containing layer (dermal tissue), and the top keratinocyte-containing layer (epithelial tissue), so that the artificial skin had a structure close to that of in vivo skin tissue (Figures 10 and 11). The keratinocytes formed an epithelium-like layer, and enucleation, which is one of morphological indicators for keratinocyte differentiation, was observed (Figure 11). In addition, the artificial skin at keratinocyte differentiation day 7 had a thickness (the distance from the surface of the epithelial tissue to the bottom surface of the bottom subcutaneous tissue) of about 4.3 mm. In view of the thickness, it was demonstrated that the thickness close to that of in vivo skin tissue was reproduced.

When the number of adipocytes used to produce an adipose tissue was changed from 1 $\times$ 10$^6$ to 5 $\times$ 10$^5$ and/or when the amount of fragmented collagen used was changed from 10 mg to 15 mg, substantially the same results as above were obtained (Figures 12 and 13). Figure 14 shows how the thickness of artificial skin with a three-layer structure correlated to the keratinocyte differentiation time. It was indicated that a decrease in the thickness of the skin was slowed over keratinocyte differentiation day 3 to 7 and was stagnated.

(Example 4: Promotion of Adipogenesis in Adipose Stem Cells in Three-dimensional Tissue)

In a fragmented collagen-containing three-dimensional tissue produced, likewise in Example 2, using human adipose stem cells and an artificial adipose tissue produced by conventional two-dimensional culture, the levels of expression of adipogenesis genes were examined by real-time quantitative polymerase chain reaction (RT-qPCR) (Figure 15).

A PureLink RNA Micro kit (manufactured by Invitrogen, Inc.) was used according to the kit protocol to extract total RNA from the above three-dimensional tissue or the artificial adipose tissue produced by conventional two-dimensional culture. The extracted RNA was quantified using Nanodrop (registered trademark) N1000 (manufactured by Thermo Fisher Scientific, Inc.). A High Capacity RNA-to-cDNA kit was used according to the kit protocol to convert 1 $\mu$g of the RNA to cDNA. In the PCR, 70 ng of the cDNA, 0.3 $\mu$M Forward and Reverse primers as well as iTaq (registered trademark) Universal SYBR Green Supermix (manufactured by Bio-Rad Laboratories, Inc.) were used for amplification. Table 1 shows each primer sequence used, the number of optimal amplification cycles in RT-PCR, and the temperature conditions. The cDNA synthesis and the RT-qPCR reaction were carried out using a StepOnePlus (registered trademark) real-time PCR system (manufactured by Thermo Fisher Scientific, Inc.).

[Table 1]

| Gene | Primer sequence (5' -> 3') | SEQ ID No. | The number of cycles | Annealing temperature (°C) |
|---|---|---|---|---|
| **PPAR$\gamma$2** | Forward: GCGATTCCTTCACTGATAC<br>Reverse: GCATTATGAGACATCCCCAC | 1<br>2 | 40 | 60 |
| **FABP4 (aP2)** | Forward: AACCTTAGATGGGGGTGTCC<br>Reverse: ATGCGAACTTCAGTCCAGGT | 3<br>4 | 40 | 57 |
| **GLUT-4** | Forward: TTCCAACAGATAGGCTCCGAAG<br>Reverse: AAGCACCGCAGAGAACACAG | 5<br>6 | 45 | 60 |
| **RPII** | Forward: CTTCACGGTGCTGGGCATT<br>Reverse: GTGCGGCTGCTTCCATAA | 7<br>8 | 40 | 60 |

During adipogenis, initial early adipogenesis genes (*e.g., FABP4, PPAR$\gamma$y2*) are expressed when preadipocytes become premature adipocytes. Then, while lipids accumulate in fat vesicles, late genes (e.g., *GLUT4*) are expressed. In this way, the growth stage is switched to the differentiation stage. The levels of expression of the early and late markers were found to gradually increase over the culturing period. However, a difference in the levels of expression was observed between the three-dimensional tissue and the artificial adipose tissue produced by two-dimensional culture. The levels of mRNA in the fragmented collagen-containing three-dimensional tissue tended to be higher than those of the artificial adipose tissue produced by two-dimensional culture. The level of expression of the early gene *FABP4* at day 7 had a maximum 5.5-fold difference and the level of expression of the late gene *GLUT4* at day 21 had a 8.3-fold difference (Figure 15).

In the artificial adipose tissue produced by two-dimensional culture, the level of expression of the PPAR$\gamma$2 gene has a continuous increase. By contrast, in the three-dimensional tissue, the expression profile in which the level increased slightly from day 7 to day 21 was seen, but the level was almost constant. In the three-dimensional tissue, *PPAR$\gamma$2* was already at a constant expression stage, and adipogenesis seemed to have progressed more. In the three-dimensional tissue, the level of another early marker *FABP4* stayed substantially the same from day 7 to day 21. By contrast, in the artificial adipose tissue produced by conventional two-dimensional culture, the level of expression was found to increase from day 7 to day 21. In the three-dimensional tissue, the level of expression of a late marker insulin-stimulated *GLUT-4* rapidly increased from day 14. By contrast, in the artificial adipose tissue produced by two-dimensional culture, it was observed that the level of expression of *GLUT-4* was very weak. These results have demonstrated that the above three-dimensional tissue has the gene expression levels reflecting differentiation states better than those of the artificial adipose tissue produced by two-dimensional culture.

**Claims**

1. An artificial adipose tissue comprising a three-dimensional tissue comprising: cells comprising an adipocyte; and a fragmented extracellular matrix.

2. The artificial adipose tissue according to claim 1, wherein the extracellular matrix comprises collagen.

3. The artificial adipose tissue according to claim 1 or 2, wherein a thickness thereof is from 1 mm to 10 mm.

4. The artificial adipose tissue according to any one of claims 1 to 3, wherein an average length of the fragmented extracellular matrix is from 100 nm to 200 $\mu$m.

**5.** An artificial skin comprising a first layer and a second layer, wherein the first layer comprises the artificial adipose tissue according to any one of claims 1 to 4.

**6.** The artificial skin according to claim 5, wherein the second layer comprises fibroblasts.

**7.** The artificial skin according to claim 5 or 6, further comprising a third layer, wherein the third layer comprises keratinocytes.

**8.** The artificial skin according to any one of claims 5 to 7, wherein a thickness thereof is from 200 $\mu$m to 10 mm.

**9.** A method for producing an artificial adipose tissue, comprising:

(1) contacting cells comprising an adipocyte with a fragmented extracellular matrix in an aqueous medium; and
(2) culturing the cells contacted with the fragmented extracellular matrix.

**10.** The production method according to claim 9, wherein the extracellular matrix comprises collagen.

**11.** The production method according to claim 9 or 10, wherein an average length of the fragmented extracellular matrix is from 100 nm to 200 $\mu$m.

**12.** A method for producing an artificial skin, comprising:

forming a first layer, comprising

(1) contacting first cells comprising an adipocyte with a first fragmented extracellular matrix in an aqueous medium, and
(2) culturing the first cells contacted with the first fragmented extracellular matrix; and

forming a second layer, comprising (3) further contacting the first layer with second cells and culturing the second cells.

**13.** The method for producing an artificial skin according to claim 12, wherein an average length of the first fragmented extracellular matrix is from 100 nm to 200 $\mu$m.

**14.** The method for producing an artificial skin according to claim 12 or 13, wherein step (3) is a step comprising contacting the second cells with a second fragmented extracellular matrix on the first layer in an aqueous medium and culturing the second cells, wherein the second extracellular matrix comprises collagen.

**15.** The method for producing an artificial skin according to any one of claims 12 to 13, wherein the second cells comprise fibroblasts.

**16.** The method for producing an artificial skin according to any one of claims 12 to 15, further comprising
forming a third layer, comprising contacting the second layer with third cells and culturing the third cells, wherein the third cells comprise keratinocytes.

**17.** An agent for culturing an adipocyte, comprising a fragmented extracellular matrix.

**18.** The agent for culturing an adipocyte according to claim 17, wherein an average length of the fragmented extracellular matrix is from 100 nm to 200 $\mu$m.

**19.** A method for evaluating skin permeability of a compound, comprising:

providing the artificial skin according to any one of claims 5 to 8;
contacting the artificial skin with a test compound;
measuring skin permeability of the test compound using the artificial skin contacted with the test compound; and
comparing between the skin permeability of the test compound and a reference value.

# Fig.1

**Fig.2**

# Fig.3

In 3D    Day 14    100 μm

In 2D    Day 14    100 μm

# Fig.4

Fig.5

# Fig.6

Adipocyte differentiation day 37

Day 37

100 μm

Adipocyte differentiation day 72

Day 72

5 μm

Fig.7

# Fig.8

**Fig.9**

Artificial skin model constructed in 9 days

Mature adipocytes in fragmented collagen gel → 24 h → Add fibroblasts → 24 h → Add keratinocytes (Keratinocytes / Fibroblasts) → 7 days of keratinocyte differentiation period → Keratinocyte-containing epithelial tissue / Fibroblast-containing dermal tissue / Adipocyte-containing subcutaneous tissue

# Fig.10

Fig.11

Fig.12

*Fig.13*

*Fig.14*

A decrease in the skin was stagnated over day 3 to day 7

Keratinocyte differentiation day（days）

—■— 10mg、5×10$^5$cells     —●— 10mg、1×10$^6$cells     —○— 15mg、1×10$^6$cells

EP 3 708 653 A1

Fig.15

EP 3 708 653 A1

## INTERNATIONAL SEARCH REPORT

| International application No. |
|---|
| PCT/JP2018/041659 |

A. CLASSIFICATION OF SUBJECT MATTER
Int.Cl. C12N5/077(2010.01)i, C12Q1/02(2006.01)i

According to International Patent Classification (IPC) or to both national classification and IPC

B. FIELDS SEARCHED

Minimum documentation searched (classification system followed by classification symbols)
Int.Cl. C12N5/077, C12Q1/02

| Documentation searched other than minimum documentation to the extent that such documents are included in the fields searched | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JST7580 (JDreamIII)
CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | SUGIHARA, H. et al., "Effects of fat cells on keratinocytes and fibroblasts in a reconstructed rat skin model using collagen gel matrix culture.", Br J Dermatol., February 2001, vol. 144, no. 2, 244-253(abstract, fig. 1-3, page 247, left column, line 3 from the bottom to page 248, left column, line 2) | 1-19 |
| Y | JP 2009-532054 A (THE OPEN UNIVERSITY) 10 September 2009, claims 1, 2, paragraph [0028] & US 2009/0221022 A1 (claims 1, 2, paragraph [0034]) & GB 2436837 A & GB 606764 D0 & WO 2007/113591 A2 & EP 2007876 A2 & CA 2648361 A & CN 101448932 A & BR PI0710522 A & IL 194524 D | 1-19 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 December 2018 (17.12.2018) | 08 January 2019 (08.01.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2018/041659 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2003-116532 A (TOYOBO BOSEKI KABUSHIKI KAISHA) 22 April 2003, claim 2 (Family: none) | 1-19 |
| Y | 松崎典弥ら，コラーゲンマイクロファイバーを用いた沈殿培養による高密度 ECM を有する 3D—結合組織の構築，高分子学会予稿集，06 September 2017, vol. 66 no. 2, ROMBUNNO.3N02 (fig. 1, <experiment>, <results and discussions>), non-official translation (MATSUSAKI Michiya et al., "Construction of 3D-connective tissue having high density ECM through sedimentation culture in which collagen microfiber is used", Proceedings of the Society of Polymer Science, Japan) | 1-19 |
| Y | 西宏基ら，コラーゲンマイクロファイバーを用いた iPS 細胞由来心筋線維化モデルの構築，高分子学会予稿集，06 September 2017, vol. 66 no. 2, ROMBUNNO.3N03 (fig. 1, <experiment>, <results and discussions>), (NISHI, Koki et al., "Construction of iPS cell-derived cardiomyocyte tissue using collagen microfiber", Proceedings of the Society of Polymer Science, Japan) | 1-19 |
| Y | JP 2002-218971 A (KANEBO LTD.) 06 August 2002, paragraphs [0002]-[0003] (Family: none) | 19 |
| Y | JP 2016-016267 A (OSAKA UNIVERSITY et al.) 01 February 2016, claim 15 (Family: none) | 19 |
| P, A | WO 2018/143286 A1 (TOPPAN PRINTING CO., LTD.) 09 August 2018, example 11 (Family: none) | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TODA S ; UCHIHASHI K ; AOKI S et al.** *Organogenesis.,* 2009, vol. 5 (2), 50-56
- **WAQAR HASSAN et al.** *STEM CELL RESEARCH & THERAPY,* 2013, vol. 4 (2), 32
- **DAQUINAG et al.** *TISSUE ENGINEERING. PART C, METHODS,* May 2013, vol. 19 (5), 336-44
- **TROTTIER et al.** *STEM CELLS,* October 2008, vol. 26 (10), 2713-23
- **MONFORT et al.** *J Tissue Eng Regen Med.,* June 2013, vol. 7 (6), 479-90
- **B. GALATEANU et al.** *Int. J. Mol. Sci.,* 2012, vol. 13, 15881-15900
- **A. SOUKAS et al.** *J. Biol. Chem.,* 2001, vol. 276, 34167-34174
- **E. C. M. MARIMAN et al.** *Cell. Mol. Life Sci.,* 2010, vol. 67, 1277-1292
- **S.-W. QIAN et al.** *BMC Dev. Biol.,* 2010, vol. 10, 47